# EUROPEAN PATENT APPLICATION

(11) **EP 4 806 378 A2**
(43) Date of publication of application: **16.09.2026**
(21) Application number: 26195969.6
(22) Date of filing: 21.12.2022
(51) Int. Cl.: A61B 34/00

(54) **SURGICAL INSTRUMENT INCLUDING ELECTRICAL AND FLUID ISOLATION FEATURES**

(30) Priority: 28.12.2021 US 202163294103 P
(62) Document number(s) of the earlier application(s) in accordance with Art. 76 EPC: 22850898.2 / 4 456 807
(71) Applicant: Intuitive Surgical Operations, Inc., Sunnyvale, CA 94086 (US)
(72) Inventor: LIMON, Timothy A., Sunnyvale, 94086 (US); WIXEY, Matthew A., Sunnyvale, 94086 (US)
(74) Representative: Mathys & Squire

(57) **Abstract**

A medical device includes a shaft, an electrically insulative outer cover, an electrically insulative spacer, and a wrist assembly. The outer cover is positioned over an outer surface of the shaft and a distal end portion of the outer cover extends beyond a distal end surface of the shaft. The spacer comprises a first coupling portion, a second coupling portion, and a circumferential annular protrusion. The first coupling portion of the spacer is coupled to an inner surface of the shaft. The wrist assembly is coupled to the second coupling portion of the spacer. The annular protrusion of the spacer includes a shoulder and a seal surface. The shoulder of the annular protrusion is in contact with the distal end surface of the shaft, and the shaft is electrically isolated by the seal surface of the annular protrusion contacting the inner surface of the outer cover.

## Description

### Related Applications

This patent application claims priority to and the filing date benefit of U.S. Provisional Patent Application No. 63/294,103, filed December 28, 2021, entitled "SURGICAL INSTRUMENT INCLUDING ELECTRICAL AND FLUID ISOLATION FEATURES," which is incorporated by reference herein in its entirety.

### Background

The embodiments described herein relate to medical devices, and more specifically to endoscopic tools. More particularly, the embodiments described herein relate to medical devices that include mechanisms for providing fluid and electrical isolation within the medical devices.

Known techniques for Minimally Invasive Surgery (MIS) employ instruments to manipulate tissue that can be either manually controlled or controlled via computer-assisted teleoperation. Many known MIS instruments include a therapeutic or diagnostic end effector (e.g., forceps, a cutting tool, or a cauterizing tool) mounted on a wrist mechanism at the distal end of a shaft, During an MIS procedure, the end effector, wrist mechanism, and the distal end of the shaft are inserted into a small incision or a natural orifice of a patient to position the end effector at a work site within the patient's body. The optional wrist mechanism can be used to change the end effector's orientation with reference to the shaft to perform the desired procedure at the work site. Known wrist mechanisms generally provide the desired mechanical degrees of freedom (DOFs) for movement of the end effector. For example, known wrist mechanisms are able to change the pitch and yaw orientation of the end effector with reference to the shaft's longitudinal axis. A wrist may optionally provide a roll DOF for the end effector with reference to the shaft, or an end effector roll DOF may be implemented by rolling the shaft, wrist, and end effector together as a unit. An end effector may optionally have additional mechanical DOFs, such as grip or knife blade motion. In some instances, wrist and end effector mechanical DOFs may be combined to provide various end effector control DOFs. For example, U.S. Patent No. 5,792,135 (filed May 16, 1997) discloses a mechanism in which wrist and end effector grip mechanical DOFs are combined to provide an end effector yaw control DOF.

To enable the desired movement of the distal wrist mechanism and end effector, known instruments include cables that extend through the shaft of the instrument and that connect the wrist mechanism to a mechanical structure configured to move the cables to operate the wrist mechanism and end effector. For teleoperated systems, the mechanical structure is typically motor driven and is operably coupled to a computer processing system to provide a user interface for a clinical user (e.g., a surgeon) to control the instrument as a whole, as well as the instrument's components and functions.

Patients benefit from continual efforts to improve the effectiveness of MIS methods and devices. For example, reducing the size and/or the operating footprint of the shaft and wrist mechanism can allow for smaller entry incisions and reduced need for space at the surgical site, thereby reducing the negative effects of surgery, such as pain, scarring, and undesirable healing time. But producing small medical devices that implement the clinically desired functions for minimally invasive procedures can be challenging. Specifically, simply reducing the size of known wrist mechanisms by scaling down the components will not result in an effective solution because required component and material properties do not scale at relatively small physical dimensions. For example, efficient implementation of a wrist mechanism can be complicated because the cables must be carefully routed through the wrist mechanism to maintain cable tension throughout the range of motion of the wrist mechanism or end effector and to minimize the interactions (coupling effects) of motion about one rotation axis upon motion about another rotation axis. As another example, pulleys and/or contoured surfaces are generally needed to reduce cable friction, which extends instrument life and permits operation without excessive forces being applied to the cables or other structures in the wrist mechanism. But increased localized forces that may result from smaller structures and cable bend radii (including smaller diameter cables and other wrist and end effector components) can result in undesirable lengthening (e.g., stretch or creep) of the cables during storage and use, reduced cable life, and the like.

Further, the wrist mechanism generally provides specific degrees of freedom for movement of the end effector. For example, for forceps or other grasping tools, the wrist may be able to change the end effector pitch, yaw, and grip orientations with reference to the instrument shaft. More degrees of freedom could be implemented through the wrist but would require additional actuation members (e.g., cables) in the wrist and shaft, and these additional members compete for the limited space that exists given the size restrictions required by MIS applications. Components needed to actuate other degrees of freedom, such as end effector roll or insertion/withdrawal through movement of the main tube, also compete for space at or in the shaft of the device.

A conventional architecture for a wrist mechanism in a manipulator-driven medical device uses cables pulled in and payed out by a capstan in the proximal mechanical structure and thereby rotate the portion of the wrist mechanism that is connected to the capstan via the cables. For example, a wrist mechanism can be operably coupled to three capstans-one each for rotations about a pitch axis, a yaw axis, and a grip axis. Each capstan can be controlled by using two cables that are attached to the capstan so that one side pays out cable while the other side pulls in an equal length of cable. With this architecture, three degrees of freedom require a total of six cables extending from the wrist mechanism proximally back along the length of the instrument's main shaft tube to the instrument's proximal mechanical structure. Efficient implementation of a wrist mechanism and proximal mechanical structure can be complicated because the cables must be carefully routed through the tool member, wrist mechanism, and proximal mechanical structure to maintain stability of the wrist throughout the range of motion of the wrist mechanism and to minimize the interactions (or coupling effects) of one rotation axis upon another.

Some known architectures for a manipulator-driven medical device include electrically charged wrist components. In such medical devices, there is a need to protect the tissue being treated from contacting the electrically charged wrist components while maintaining flexibility of the wrist. In such devices, it can be challenging preventing electrically conductive fluid, such as body fluid, from pooling and capacitively coupling with applied energy. In addition, some known medical devices may include a shaft constructed with electrically conductive material such as stainless steel to reduce cost and increase strength. For example, in some known medical devices, the shaft bears a high axial load within the system and presents challenges in providing the strength needed to bear the load while simultaneously meeting the needed size limitations for the shaft and maintaining cost effectiveness. In such devices, however, the need to eliminate electrically conductive fluid contact with the shaft is increased to prevent unintended tissue damage from electrical charge.

Thus, a need exists for improved endoscopic tools that provide a mechanical structure with fluid and electrical isolation features at or near the distal end of the endoscopic tool to facilitate the use of an electrically conductive shaft, such as a stainless steel shaft, by accommodating inner and outer insulation of the shaft and preventing fluid leakage.

A need also exists for improved endoscopic tools, including improved proximal mechanical structures to provide for axial strength of the shaft and coupling and locking of an outer electrical insulation cover about the electrically conductive shaft to preserve fluid sealing at the distal end of the endoscopic tool.

### Summary

This summary introduces certain aspects of the embodiments described herein to provide a basic understanding. This summary is not an extensive overview of the inventive subject matter, and it is not intended to identify key or critical elements or to delineate the scope of the inventive subject matter.

In some embodiments, a medical device includes a shaft, an electrically insulative outer cover, an electrically insulative spacer, and a wrist assembly. The shaft comprises an outer surface, an inner surface, and a distal end surface. The outer cover is positioned over the outer surface of the shaft and comprises an inner surface and a distal end portion. The distal end portion of the outer cover extends beyond the distal end surface of the shaft. The spacer comprises a first coupling portion, a second coupling portion, and a circumferential annular protrusion. The first coupling portion of the spacer is coupled to the inner surface of the shaft. The wrist assembly is coupled to the second coupling portion of the spacer. The annular protrusion of the spacer comprises a shoulder and a seal surface. The shoulder of the annular protrusion is in contact with the distal end surface of the shaft, and the shaft is electrically isolated by the seal surface of the annular protrusion contacting the inner surface of the outer cover. In some embodiments, at least a portion of the shaft between the outer cover and the inner cover is electrically conductive.

In some embodiments, the shoulder of the annular protrusion is a first shoulder, the spacer comprises a second shoulder and a mounting recess between the annular protrusion and the second shoulder and the medical device further comprises a tip cover. The tip cover comprises a coupling protrusion and a first seal surface. The coupling protrusion is within the mounting recess, and fluid is prevented from passing between the tip cover and the spacer by the first seal surface of the tip cover contacting the second shoulder of the spacer.

In some embodiments, the distal end portion of the outer cover extends over the mounting recess of the spacer to enclose the coupling protrusion of the tip cover. The tip cover comprises a second seal surface, and fluid is prevented from passing between the tip cover and the outer cover by a seal formed between a distal end surface of the outer cover and the second seal surface.

In some embodiments, the tip cover comprises an inner cover member and an outer cover member. The inner cover member is constructed from a first material having a hardness, and the outer cover member is constructed from a second material having a hardness. The hardness of the first material is more than the hardness of the second material.

In some embodiments, the spacer comprises a spacer passageway, the second coupling portion of the spacer comprises a plurality of protrusions within the spacer passageway, and the wrist assembly is coupled to the spacer by the plurality of protrusions engaging the wrist assembly. In some embodiments, the medical device includes a cable and the spacer comprises a cable opening. The cable is routed from the shaft through the cable opening of the spacer to the wrist assembly. In some embodiments, the wrist assembly comprises a proximal link and the proximal link comprises a coupling protrusion. The wrist assembly is coupled to the spacer by the plurality of protrusions of the second coupling portion of the spacer engaging the coupling protrusion.

In some embodiments, the medical device comprises an electrically conductive proximal link and the proximal link comprises a coupling protrusion. The coupling protrusion of the proximal link comprises an electrical connector configured to receive an electrical wire to electrically couple the electrical wire to the proximal link.

In some embodiments, the spacer comprises a third coupling portion and the medical device further comprises an insulation member adjacent the inner surface of the shaft. The insulation member is coupled to the third coupling portion of the spacer. In some embodiments, the spacer comprises an inner surface and a spacer passageway. The wrist assembly comprises a coupling protrusion within the spacer passageway and the medical device further comprises a seal and a cable. The seal comprises a cable seal opening and is coupled to the coupling protrusion of the wrist within the spacer passageway to form a seal between the coupling protrusion of the wrist and the inner surface of the spacer. The cable is routed from the shaft, through the cable opening of the seal, and to the wrist assembly. In some embodiments, the cable has an untensioned diameter in an untensioned state of the cable, and the cable seal opening has a diameter less than the untensioned diameter of the cable.

In some embodiments, the shaft comprises a proximal end portion and the medical device further comprises a proximal mechanical structure. An electronic circuit is located within the proximal mechanical structure. The proximal end portion of the shaft is coupled to the proximal mechanical structure and the shaft is electrically coupled to the electronic circuit. In some embodiments, the electronic circuit comprises a processor. The processor is configured to receive an input signal associated with a change of impedance of the shaft and to produce a control signal on a condition the input signal received by the processor indicates an electrically conductive breach in the outer cover.

In some embodiments, a medical device includes a shaft comprising a distal end portion, an outer surface, a first shoulder, a second shoulder, and a mounting recess defined by the outer surface between the first shoulder and the second shoulder. The medical device further includes an outer cover having a distal end surface, and a wrist assembly that is coupled to the distal end portion of the shaft. The medical device further includes a tip cover comprises an outer surface, a coupling protrusion, a first seal surface, and a second seal surface. The outer cover surrounds the outer surface of the shaft. The outer surface of the tip cover surrounds the wrist assembly. The coupling protrusion of the tip cover is within the mounting recess of the shaft between the outer cover and the shaft such that the first seal surface of the tip cover forms a first seal with the second shoulder of the shaft, and the second seal surface of the tip cover forms a second seal with the distal end surface of the outer cover.

In some embodiments, the tip cover comprises an inner cover member and an outer cover member. The inner cover member is constructed from a first material having a hardness, the outer cover member is constructed from a second material having a hardness, and the hardness of the first material is more than the hardness of the second material.

In some embodiments, the shaft comprises a passageway and a set of protrusions within the passageway. The wrist assembly is coupled to the shaft by the set of protrusions engaging the wrist assembly. In some embodiments, the medical device includes a cable and the shaft comprises a cable opening. The cable is routed through the passageway of the shaft, through the cable opening of the shaft and to the wrist assembly.

In some embodiments, the wrist assembly comprises an electrically conductive proximal link. The proximal link comprises a link coupling protrusion and the link coupling protrusion of the proximal link comprises an electrical connector configured to receive an electrical wire to electrically couple the electrical wire to the proximal link.

In some embodiments, the shaft comprises a coupling portion and the medical device further comprises an insulation member adjacent to the inner surface of the shaft. The insulation member is coupled to the coupling portion of the shaft.

In some embodiments, a medical device includes a shaft including a proximal end portion, a distal end portion, an inner surface, and a passageway defined by the inner surface. A seal is coupled within the passageway of the shaft, and the cable seal includes a cable seal opening. A link is coupled to the distal end portion of the shaft and a tool member is rotatably coupled to the link. A cable including a proximal end portion and a distal end portion is coupled to the tool member. The proximal end portion of the cable is routed within the passageway of the shaft and the distal end portion of the cable is routed through the cable seal opening of the seal and is coupled to the tool member. Movement of the cable causes rotation of the tool member. The distal end portion of the cable has a first diameter when the cable is in an untensioned state and a second diameter when the cable is in a tensioned state. The cable seal opening has a diameter. The first diameter of the cable is greater than the diameter of the cable seal opening, and the second diameter of the cable is less than the diameter of the cable seal opening.

In some embodiments, the cable is a polymeric braided construction. In some embodiments, at least one of the distal end portion of the cable or a portion of the seal that defines the cable seal opening includes an oil coating. In some embodiments, at least one of the distal end portion of the cable or a portion of the seal that defines the cable seal opening comprises a hydrophobic material. In some embodiments, the cable seal opening includes a first taper portion, a second taper portion, and a throat that defines the diameter of the cable seal opening. In some embodiments, a ratio between the first diameter and the second diameter of the cable is between 1.2:1 and 1.6:1.

In some embodiments, the seal defines an outer seal portion that forms a seal within the inner surface of the shaft. In some embodiments, the link includes a proximal coupling protrusion positioned within the passageway of the shaft and the seal defines an inner seal portion that forms a seal within the coupling protrusion.

In some embodiments, a medical device includes a chassis component comprising a distal surface and an opening defined in the distal surface. A shaft extends into the opening of the chassis component and is coupled to the chassis component. The medical device further includes an outer cover having a proximal end portion and a flange positioned at least partially within the chassis component. A lock ring is positioned about the flange within the opening of the chassis component. The outer cover at least partially surrounds the shaft and the proximal end portion of outer cover extends into the opening of the chassis component. The flange comprises a shaft coupling portion and a plurality of locking teeth. The shaft coupling portion of the flange is coupled to the shaft. The lock ring is positioned to urge the locking teeth to engage the proximal end portion of the outer cover to fixedly couple the outer cover to the shaft.

In some embodiments, the flange comprises a proximal end, a distal end, a centerline defined between the proximal end and the distal end, a first portion, and a second portion. The first portion of the flange is coupled to the second portion of the flange along a plane that includes the centerline of the flange. In some embodiments, a portion of the flange that comprises the plurality of locking teeth is deformable. In some embodiments, the shaft comprises a first opening and a second opening. The shaft coupling portion of the flange comprises a first boss and a second boss. The first boss of the flange is received in the first opening of the shaft and the second boss of the flange is received in the second opening of the shaft.

In some embodiments, the medical device further comprises a roll gear. The flange comprises a roll gear mounting portion coupled to the roll gear and rotation of the roll gear rotates the shaft and the flange relative to the chassis component. In some embodiments, the medical device further comprises a bearing coupled within the chassis component, and the flange comprises a bearing mounting portion coupled to the bearing.

In some embodiments, the lock ring comprises a distal portion that has an outer diameter larger than the opening of the chassis component, and the distal portion of the lock ring is outside of the chassis component.

Other medical instruments, related components, medical device systems, and/or methods according to embodiments will be or become apparent to one with skill in the art upon review of the following drawings and detailed description. It is intended that all such additional medical devices, related components, medical device systems, and/or methods included within this description be within the scope of this disclosure.

### Brief Description of the Drawings

FIG. 1 is a plan view of a minimally invasive teleoperated medical system according to an embodiment being used to perform a medical procedure such as surgery.
FIG. 2 is a perspective view of an optional auxiliary unit of the minimally invasive teleoperated surgery system shown in FIG. 1.
FIG. 3 is a perspective view of a user control console of the minimally invasive teleoperated surgery system shown in FIG. 1.
FIG. 4 is a front view of a manipulator unit, including a plurality of instruments, of the minimally invasive teleoperated surgery system shown in FIG. 1.
FIG. 5A is a diagrammatic illustration of a portion of a medical device according to an embodiment.
FIG. 5B is an enlarged view of circled portion Z in FIG. 5A.
FIG. 5C is a schematic illustration of a portion of a cable, shown in a first untensioned configuration.
FIG. 5D is a schematic illustration of the portion of the cable of FIG. 5C, shown in a second tensioned configuration.
FIG. 6 is a schematic illustration of a distal end portion of a medical device according to an embodiment.
FIG. 7 is a schematic illustration of a distal end portion of a medical device according to an embodiment.
FIG. 8 is a schematic illustration of a proximal end portion of a medical device according to an embodiment.
FIG. 9 is a perspective view of a medical device according to an embodiment.
FIG. 10 is a perspective view of a distal end portion of the medical device of FIG. 9 with a distal tip cover and outer cover removed.
FIG. 11 is an exploded side view of select components of a distal end portion of the medical device of FIG. 9.
FIG. 12 is a perspective view of a distal end portion of the medical device of FIG. 9, shown with the outer cover in an advanced (i.e., distal) position.
FIG. 13 is a cross-sectional perspective view of a distal end portion of the medical device of FIG. 9, shown with the outer cover in the advanced position and the tool member of the end effecter removed for illustrative purposes.
FIG. 14A is an enlarged view of a portion of the cross-sectional perspective view of a distal end portion of the medical device of FIG. 13.
FIG. 14B is an enlarged view of a portion of the cross-sectional perspective view of a distal end portion of the medical device of FIG. 14A.
FIG. 14C is an enlarged view of a portion of the cross-sectional perspective view of a distal end portion of the medical device of FIG. 14A, illustrating example distance dimensions between select components.
FIG. 14D is an enlarged view of a portion of the cross-sectional perspective view of a distal end portion of the medical device of FIG. 14A, illustrating example potential leak paths for liquids and/or gases to pass.
FIG. 15A is a partial exploded perspective view of select components of a distal end portion of the medical device of FIG. 9.
FIG. 15B is a distal perspective view of a portion of the spacer and the seal of the medical device of FIG. 9.
FIG. 15C is a partial exploded perspective view of select components of a distal end portion of the medical device of FIG. 9.
FIG. 16A is a perspective distal view of a seal of the medical device of FIG. 9.
FIG. 16B is a distal end view of the seal of the medical device of FIG. 9.
FIG. 16C is a perspective cross-sectional view of the seal of the medical device of FIG. 9, illustrating the inner cable openings of the seal.
FIG. 16D is a side cross-sectional view of the seal of the medical device of FIG. 9, illustrating the inner cable openings of the seal.
FIG. 17 is a cross-sectional view of a portion of a distal end of a medical device according to an embodiment having an alternative distal tip cover.
FIGS. 18A and 18B are each a cross-sectional perspective view of a different portion of a distal end of a medical device according to an embodiment having another alternative distal tip cover.
FIG. 19 is a perspective view of the distal end portion of the medical device of FIG. 9, shown with the outer cover in a retracted (i.e., proximal) position.
FIG. 20 is a cross-sectional perspective view of a distal end portion of the medical device of FIG. 9, shown with the outer cover in the retracted position and the tool member of the end effecter removed for illustrative purposes.
FIG. 21 is an enlarged view of a portion of the cross-sectional perspective view of a distal end portion of the medical device of FIG. 20.
FIG. 22 is a perspective view of a portion of the proximal mechanical structure of the medical device of FIG. 9 with select components removed for illustration purposes.
FIG. 23 is a side perspective view of a portion of the mechanical structure of the medical device of FIG. 9 with select components removed for illustration purposes.
FIG. 24 is a cross-sectional perspective view of a portion of the mechanical structure of the medical device of FIG. 9, with the outer cover in the advanced (i.e., distal) position.
FIG. 25 is an enlarged cross-sectional perspective view of a portion of the mechanical structure of the medical device of FIG. 9, with the outer cover in the advanced position.
FIG. 26A is an enlarged cross-sectional perspective view of a portion of the mechanical structure of the medical device of FIG. 9, with the outer cover in the retracted (i.e., proximal) position.
FIG. 26B is an enlarged cross-sectional perspective view of a distal end portion of the medical device of FIG. 9, with the outer cover in the retracted position.
FIG. 27A is an enlarged cross-sectional perspective view of a portion of the mechanical structure of the medical device of FIG. 9, with the outer cover in the advanced (i.e., distal) position and with the lock ring removed.
FIG. 27B is an enlarged cross-sectional perspective view of a distal end portion of the medical device of FIG. 9, with the outer cover in the advanced position.
FIG. 28 is an enlarged cross-sectional perspective view of a portion of the mechanical structure of the medical device of FIG. 9, with the outer cover in the advanced position and with the lock ring coupled to the flange.
FIG. 29 is an enlarged cross-sectional perspective view of a portion of the mechanical structure of the medical device of FIG. 9, with the outer cover in the advanced position and with the lock ring coupled to the flange illustrating locking teeth engaging the outer cover.
FIG. 30 is an enlarged cross-sectional perspective view of a portion of the mechanical structure of the medical device of FIG. 9, with the outer cover in the advanced position, illustrating the lock ring engaged with a protrusion of the flange.
FIG. 31A is a perspective view of the flange and shaft of the medical device of FIG. 9.
FIG. 31B is an exploded view of the flange of FIG. 31A.
FIG. 32 is a perspective view of select components of the mechanical structure and a portion of the shaft of the medical device of FIG. 9.
FIG. 33 is an exploded perspective view of the select components of the mechanical structure of FIG. 32.
FIG. 34 is a perspective view of the components of the mechanical structure of FIG. 32 with the cable guide removed for illustration purposes.
FIGS. 35A and 35B are each a different exploded perspective view of select components of the mechanical structure of the medical device of FIG. 9, illustrating an electronic circuit of the medical device.
FIGS. 36A and 36B are a perspective view of an electronic circuit of the medical device of FIG. 9 (FIG. 36A) and a partial exploded view of the electronic circuit (FIG. 36B).
FIG. 37 is a perspective view of select components of the mechanical structure of the medical device of FIG. 9 illustrating the connection of the electronic circuit to the shaft of the medical device.
FIG. 38 is a perspective view of select components of the mechanical structure of the medical device of FIG. 9 illustrating the connection of the electronic circuit to the wrist assembly of the medical device.

### DETAILED DESCRIPTION

The embodiments described herein can advantageously be used in a wide variety of grasping, cutting, and manipulating operations associated with minimally invasive surgery. In some embodiments, an end effector of the medical device can move with reference to the main body of the instrument in three mechanical DOFs, e.g., pitch, yaw, and roll (shaft roll). There may also be one or more mechanical DOFs in the end effector itself, e.g., two jaws, each rotating with reference to a clevis (2 DOFs) and a distal clevis that rotates with reference to a proximal clevis (one DOF).

The medical devices of the present application enable motion in three degrees of freedom (e.g., about a pitch axis, a yaw axis, and a grip axis) using only four cables, thereby reducing the total number of cables required, reducing the space required within the shaft and wrist, reducing overall cost, and enables further miniaturization of the wrist and shaft assemblies to promote MIS procedures. Moreover, the instruments described herein include one or more cables (which function as tension members) that are made of a polymer material and that can be secured to a capstan of the proximal end mechanism without the need for a retention element or other securing feature.

Medical devices described herein can provide for electrical power to be transferred to a wrist assembly and/or tool coupled to the wrist assembly at a distal end of the medical device, such as, for example, a cauterizing or cutting tool. The medical devices have an architecture that provides for protection of tissue from contact with the electrically charged wrist components at the distal end of the medical device, while maintaining flexibility of the wrist. In some embodiments described herein, a medical has a metallic shaft which provides for strength and reduced costs. Such medical devices have an architecture that reduces (and in some instances eliminates) fluid contact with the metallic shaft. For example, a metallic shaft can become capacitively coupled and charged during the application of energy and must be insulated to avoid unintended tissue damage. In some embodiments, the shaft can be electrically monitored for breaches in this insulation as described herein. Fluid contact from the wrist could interfere with the monitored signal and may be misinterpreted as an insulation breach. Thus, it is desirable to prevent such fluid contact. In some embodiments, the medical devices described herein include a tip cover that is positioned over the wrist assembly, and architecture on both a distal and proximal end of the medical device for securing the tip cover to the instrument. The tip cover can be formed with an insulative material to protect tissue from contact with the electrically charged wrist components at the distal end of the medical device.

As described herein, in some embodiments, a medical device includes a seal at distal end portion of the instrument that protects against escaping insufflation pressure and minimizes fluid leakage around the drive cables of the medical device without excessive friction. The seal can also prevent body fluids from reaching the electrically charged wrist components and metallic shaft.

As used herein, the term "about" when used in connection with a referenced numeric indication means the referenced numeric indication plus or minus up to 10 percent of that referenced numeric indication. For example, the language "about 50" covers the range of 45 to 55. Similarly, the language "about 5" covers the range of 4.5 to 5.5.

As used in this specification and the appended claims, the word "distal" refers to direction towards a work site, and the word "proximal" refers to a direction away from the work site. Thus, for example, the end of a medical device that is closest to the target tissue would be the distal end of the medical device, and the end opposite the distal end (i.e., the end manipulated by the user or coupled to the actuation shaft) would be the proximal end of the medical device.

Further, specific words chosen to describe one or more embodiments and optional elements or features are not intended to limit the invention. For example, spatially relative terms-such as "beneath", "below", "lower", "above", "upper", "proximal", "distal", and the like-may be used to describe the relationship of one element or feature to another element or feature as illustrated in the figures. These spatially relative terms are intended to encompass different positions (i.e., translational placements) and orientations (i.e., rotational placements) of a device in use or operation in addition to the position and orientation shown in the figures. For example, if a device in the figures is turned over, elements described as "below" or "beneath" other elements or features would then be "above" or "over" the other elements or features. Thus, the term "below" can encompass both positions and orientations of above and below. A device may be otherwise oriented (e.g., rotated 90 degrees or at other orientations) and the spatially relative descriptors used herein interpreted accordingly. Likewise, descriptions of movement along (translation) and around (rotation) various axes includes various spatial positions and orientations. The combination of a body's position and orientation define the body's pose.

Similarly, geometric terms, such as "parallel", "perpendicular", "round", or "square", are not intended to require absolute mathematical precision, unless the context indicates otherwise. Instead, such geometric terms allow for variations due to manufacturing or equivalent functions. For example, if an element is described as "round" or "generally round," a component that is not precisely circular (e.g., one that is slightly oblong or is a many-sided polygon) is still encompassed by this description.

In addition, the singular forms "a", "an", and "the" are intended to include the plural forms as well, unless the context indicates otherwise. The terms "comprises", "includes", "has", and the like specify the presence of stated features, steps, operations, elements, components, etc. but do not preclude the presence or addition of one or more other features, steps, operations, elements, components, or groups.

Unless indicated otherwise, the terms apparatus, medical device, medical instrument, and variants thereof, can be interchangeably used.

Aspects of the invention are described primarily in terms of an implementation using a *da Vinci*^{®} surgical system, commercialized by Intuitive Surgical, Inc. of Sunnyvale, California. Examples of such surgical systems are the *da Vinci Xi*^{®} surgical system (Model IS4000), *da Vinci X*^{®} Surgical System (Model IS4200), and the *da Vinci Si*^{®} surgical system (Model IS3000). Knowledgeable persons will understand, however, that inventive aspects disclosed herein may be embodied and implemented in various ways, including computer-assisted, non-computer-assisted, and hybrid combinations of manual and computer-assisted embodiments and implementations. Implementations on *da Vinci^{®}* surgical systems (e.g., the Model IS4000, the Model IS3000, the Model IS2000, the Model IS1200, the Model SP1099) are merely presented as examples, and they are not to be considered as limiting the scope of the inventive aspects disclosed herein. As applicable, inventive aspects may be embodied and implemented in both relatively smaller, hand-held, hand-operated devices that are not mechanically grounded in a world reference frame and relatively larger systems that have additional mechanical support that is grounded in a world reference frame.

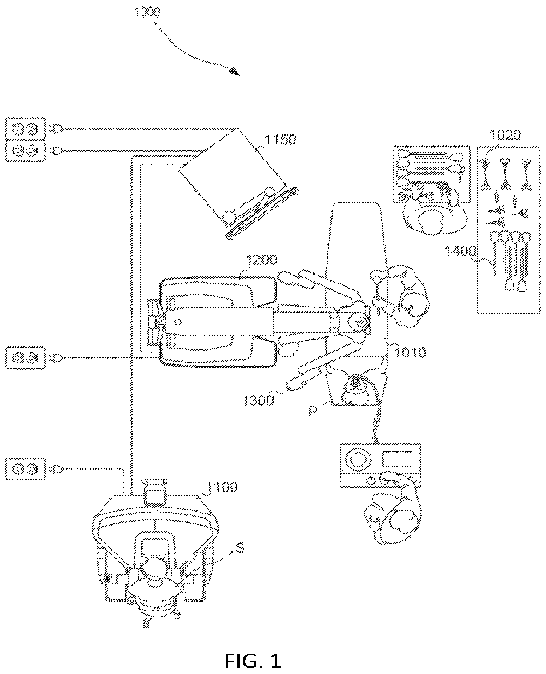

FIG. 1 is a plan view illustration of a teleoperated surgical system 1000 that operates with at least partial computer assistance (a "telesurgical system"). Both telesurgical system 1000 and its components are considered medical devices. Telesurgical system 1000 is a Minimally Invasive Robotic Surgical (MIRS) system used for performing a minimally invasive diagnostic or surgical procedure on a Patient P who is lying on an Operating table 1010. The system can have any number of components, such as a user control unit 1100 for use by a surgeon or other skilled clinician S during the procedure. The MIRS system 1000 can further include a manipulator unit 1200 (popularly referred to as a surgical robot) and an optional auxiliary equipment unit 1150. The manipulator unit 1200 can include an arm assembly 1300 and a surgical instrument tool assembly removably coupled to the arm assembly. The manipulator unit 1200 can manipulate at least one removably coupled instrument 1400 through a minimally invasive incision in the body or natural orifice of the patient P while the surgeon S views the surgical site and controls movement of the instrument 1400 through control unit 1100. An image of the surgical site is obtained by an endoscope (not shown), such as a stereoscopic endoscope, which can be manipulated by the manipulator unit 1200 to orient the endoscope. The auxiliary equipment unit 1150 can be used to process the images of the surgical site for subsequent display to the Surgeon S through the user control unit 1100. The number of instruments 1400 used at one time will generally depend on the diagnostic or surgical procedure and the space constraints within the operating room, among other factors. If it is necessary to change one or more of the instruments 1400 being used during a procedure, an assistant removes the instrument 1400 from the manipulator unit 1200 and replaces it with another instrument 1400 from a tray 1020 in the operating room. Although shown as being used with the instruments 1400, any of the instruments described herein can be used with the MIRS 1000.

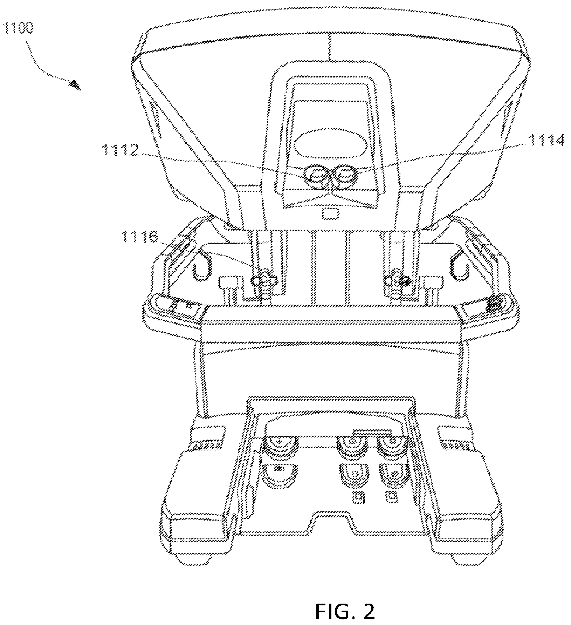

FIG. 2 is a perspective view of the control unit 1100. The user control unit 1100 includes a left eye display 1112 and a right eye display 1114 for presenting the surgeon S with a coordinated stereoscopic view of the surgical site that enables depth perception. The user control unit 1100 further includes one or more input control devices 1116, which in turn cause the manipulator unit 1200 (shown in FIG. 1) to manipulate one or more tools. The input control devices 1116 provide at least the same degrees of freedom as instruments 1400 with which they are associated to provide the surgeon S with telepresence, or the perception that the input control devices 1116 are integral with (or are directly connected to) the instruments 1400. In this manner, the user control unit 1100 provides the surgeon S with a strong sense of directly controlling the instruments 1400. To this end, position, force, strain, or tactile feedback sensors (not shown) or any combination of such sensations, from the instruments 1400 back to the surgeon's hand or hands through the one or more input control devices 1116.

The user control unit 1100 is shown in FIG. 1 as being in the same room as the patient so that the surgeon S can directly monitor the procedure, be physically present if necessary, and speak to an assistant directly rather than over the telephone or other communication medium. In other embodiments, however, the user control unit 1100 and the surgeon S can be in a different room, a completely different building, or other location remote from the patient, allowing for remote surgical procedures.

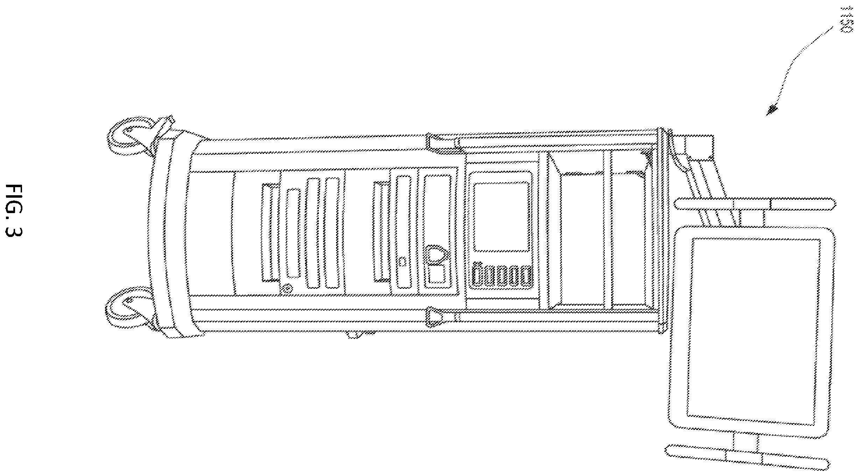

FIG. 3 is a perspective view of the auxiliary equipment unit 1150. The auxiliary equipment unit 1150 can be coupled with the endoscope (not shown) and can include one or more processors to process captured images for subsequent display, such as via the user control unit 1100, or on another suitable display located locally (e.g., on the unit 1150 itself as shown, on a wall-mounted display) and/or remotely. For example, where a stereoscopic endoscope is used, the auxiliary equipment unit 1150 can process the captured images to present the surgeon S with coordinated stereo images of the surgical site via the left eye display 1112 and the right eye display 1114. Such coordination can include alignment between the opposing images and can include adjusting the stereo working distance of the stereoscopic endoscope. As another example, image processing can include the use of previously determined camera calibration parameters to compensate for imaging errors of the image capture device, such as optical aberrations.

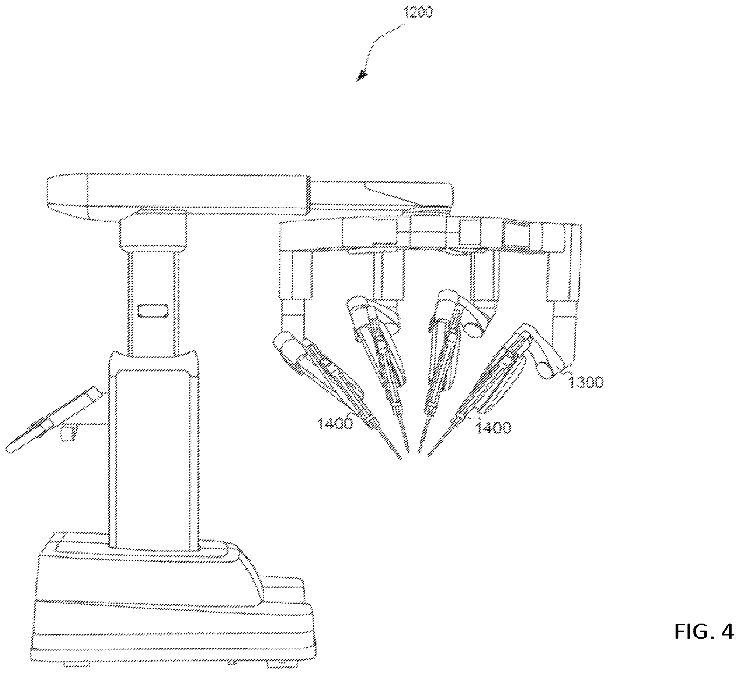

FIG. 4 shows a front perspective view of the manipulator unit 1200. The manipulator unit 1200 includes the components (e.g., arms, linkages, motors, sensors, and the like) to provide for the manipulation of the instruments 1400 and an imaging device (not shown), such as a stereoscopic endoscope, used for the capture of images of the site of the procedure. Specifically, the instruments 1400 and the imaging device can be manipulated by teleoperated mechanisms having one or more mechanical joints. Moreover, the instruments 1400 and the imaging device are positioned and manipulated through incisions or natural orifices in the patient P in a manner such that a center of motion remote from the manipulator and typically located at a position along the instrument shaft is maintained at the incision or orifice by either kinematic mechanical or software constraints. In this manner, the incision size can be minimized.

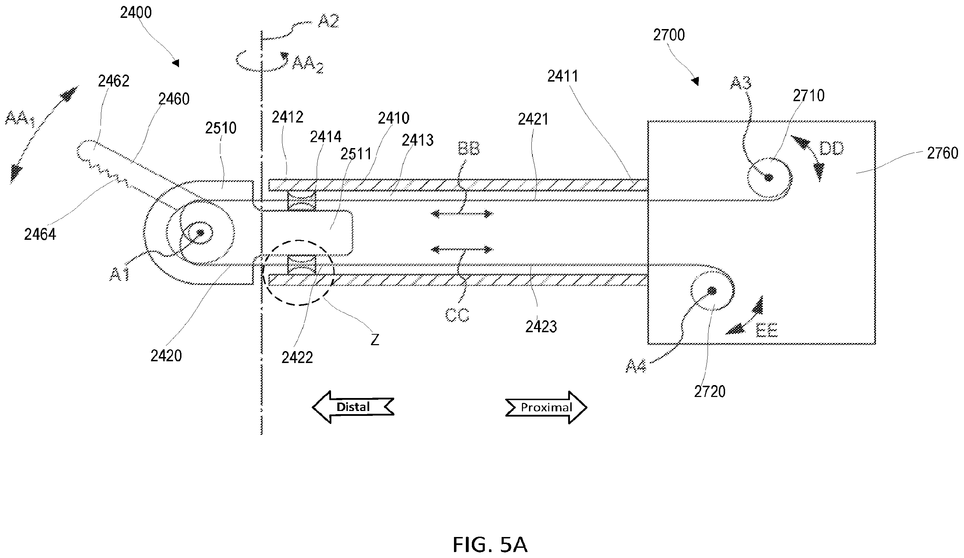

FIGS. 5A-5D are schematic illustrations of a portion of a medical device 2400 according to an embodiment. In some embodiments, the instrument 2400 or any of the components therein are optionally parts of a surgical system that performs surgical procedures, and which can include a manipulator unit, a series of kinematic linkages, a series of cannulas, or the like. The medical device 2400 (and any of the instruments described herein) can be used in any suitable surgical system, such as the MIRS system 1000 shown and described above. The medical device 2400 includes a shaft 2410, a cable 2420 (which acts as a first tension member), a seal 2414, a link 2510, an end effector 2460, and a mechanical structure 2700. In many embodiments, mechanical structure 2700 functions to receive one or more motor input forces or torques and mechanically transmit the received forces or torques to move an associated one or more components in medical device 2400. For example, one or more electric motors in manipulator unit 1200 (described above) provide an input to mechanical structure 2700, which in turn transmits the input to control a medical device 2400 component. In some optional embodiments, mechanical structure 2700 includes one or more motors used to control a medical device 2400 component. In some embodiments, the mechanical structure 2700 (and any of the mechanical structures described herein) can include one or more drive motors to produce the force or torque to move the components of the medical device 2400. In other embodiments, mechanical structure 2700 (and any of the mechanical structures described herein) is devoid of any motors and transmits force or torque from outside the medical device to one or more of the medical device's components. For example, in some embodiments, the mechanical structure 2700 (and any of the mechanical structures described herein) is coupled to a manipulator unit that includes one or more motors that drive an instrument component. The shaft 2410 includes a proximal end portion 2411 and a distal end portion 2412 and defines a passageway 2413 that extends lengthwise through the shaft between the proximal and distal end portions.

The seal 2414 is positioned within the passageway 2413 of the shaft 2410 at the distal end portion 2412 of the shaft 2410. The seal 2414 can be formed with, for example, *Santoprene*^{™} thermoplastic vulcanizates (TPVs), a thermoplastic polyurethane (TPU) material or other suitable material and can be coupled (either directly or indirectly) to an inner surface of the shaft 2410 by a friction fit, adhesive or other suitable coupling methods. The seal 2414 includes one or more cable seal openings 2416 through which the cable 2420 can pass. More specifically, the cable 2420 includes a first end, a second end, a first proximal portion 2421, a second proximal portion 2423 and a distal portion 2422. The first end and the second end are each coupled to the mechanical structure 2700, the first proximal portion 2421 and the second proximal portion 2423 extend through a passageway 2413 of the shaft 2410, and the distal portion 2422 extends through the cable seal openings 2416 and is coupled to the end effector 2460 as described in more detail below.

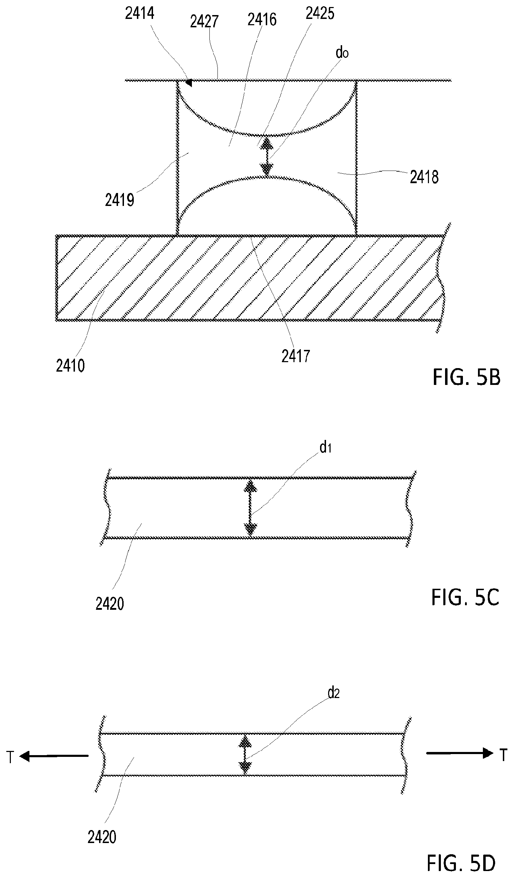

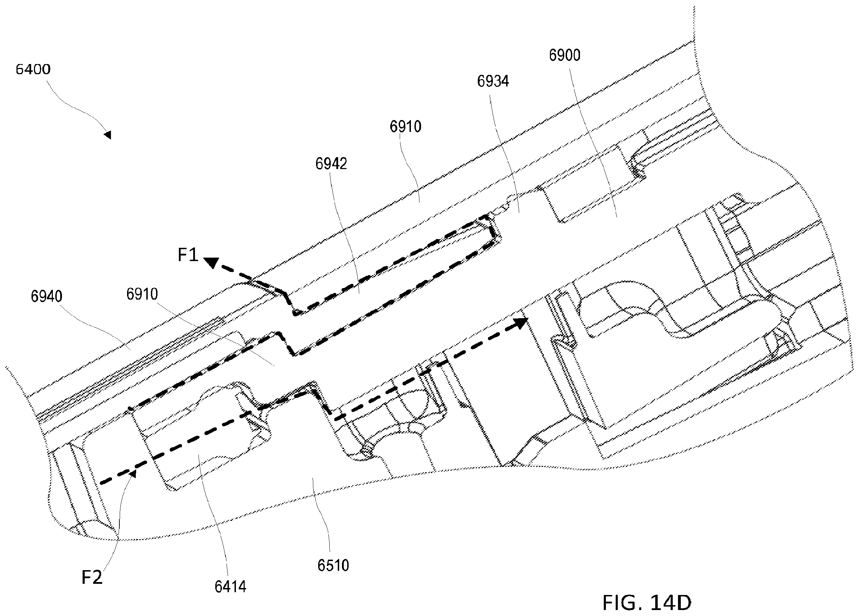

Referring to FIG. 5B, the seal 2414 defines an outer seal portion 2417 and an inner seal portion 2427 to limit liquid leakage into the shaft 2410, and also limit leakage of insufflation gas out of the interior body cavity of the patient being treated via the medical device 2400. The outer seal portion 2417 provides a seal between an inner surface of the shaft 2410 and an outer perimeter of the seal 2414, and the inner seal portion 2427 provides a seal between an outer surface of a proximal coupling protrusion 2511 of the link 2510 and an inner surface of the seal 2414. This arrangement seals the fluid path F2 shown in FIG. 14D and described below.

The cable seal openings 2416 are shaped and sized to also limit liquids and/or insufflation gas from leaking through the openings 2416 while also allowing the cable 2420 to move within the openings 2416. More specifically, as shown in FIG. 5B, the cable seal openings 2416 have an hourglass shape that has a first tapered portion 2418 at a first end of the openings 2416, a second tapered portion 2419 at a second end, and a throat portion 2425 having a smaller diameter d₀ than a diameter of the first tapered portion 2418 and the second tapered portion 2419. The hourglass shape of the cable seal openings 2416 and the diameter d₀ provide for a tight fit to the cable 2420 when the cable is under tension to prevent liquids and/or gas leakage through the openings 2416. For example, the cable 2420 can be formed with a polymeric material such that the cable 2420 can deform under tension. As shown in FIG. 5B, the cable 2420 has a first nominal or untensioned state that has a diameter d₁ and a second, tensioned state with a diameter d₂, as shown in FIG. 5D. The ratio between the diameter d₁ and the diameter d₂ can be, for example, 1.2:1 and 1.6:1. In some embodiments, the diameter of the cable 2420 can be reduced between 0 - 30% of its first nominal diameter. The diameter d₁ is larger than the diameter d₀ of the cable seal openings 2416, and the diameter d₂ of the cable 2410 is smaller than the diameter d₀. Thus, the cable 2420 can be passed through the cable seal openings 2416 when in the tensioned state and when the cable 2420 is untensioned, the cable 2420 will return to its untensioned, nominal diameter d₁ and form a tight fit to the throat portion 2425 of the cable seal openings 2416. The cable seal openings 2416 are configured to protect against escaping insufflation pressure and minimize fluid leakage around the cables 2420 of the medical device without excessive friction.

In some embodiments, the cable 2420 can be a polymeric braided construction. In some embodiments, a distal end portion of the cable 2420 and/or a portion of the seal 2414 that defines the cable seal openings 2416 can include an oil coating. In some embodiments, a distal end portion of the cable 2420 and/or a portion of the seal 2414 that defines the cable seal openings 2416 can include a hydrophobic material.

In some embodiments, the medical device 2400 can include a spacer (not shown in FIG. 5A), such as spacer 3900 and 6900, and an outer cover (not shown in FIG. 5A), such as outer cover 3910, 4910 5910 and 6910 described herein. The spacer can be positioned within the passageway 2413 of the shaft 2410 and extend distally of the shaft 3410, and the seal 2414 can be positioned within an interior passageway at a distal end of the spacer. The seal 2414 can function in the same manner as described above and provide a first sealing portion to the inner surface of the spacer and a second sealing portion via the cable seal openings 2416. The outer cover can surround the exterior of the shaft 2410 and extend distally of the distal end of the shaft 2410. In this manner, the seal 2414, the spacer and outer cover can isolate the conductive shaft 2414 from electrical energy. For example, the shaft 2410 can become capacitively coupled and charged during the application of energy, and the spacer, outer cover and seal 2414 can isolate the shaft 2414 to prevent fluid contact with the shaft 2410 and to avoid unintended damage to tissue during treatment using the medical device 2400.

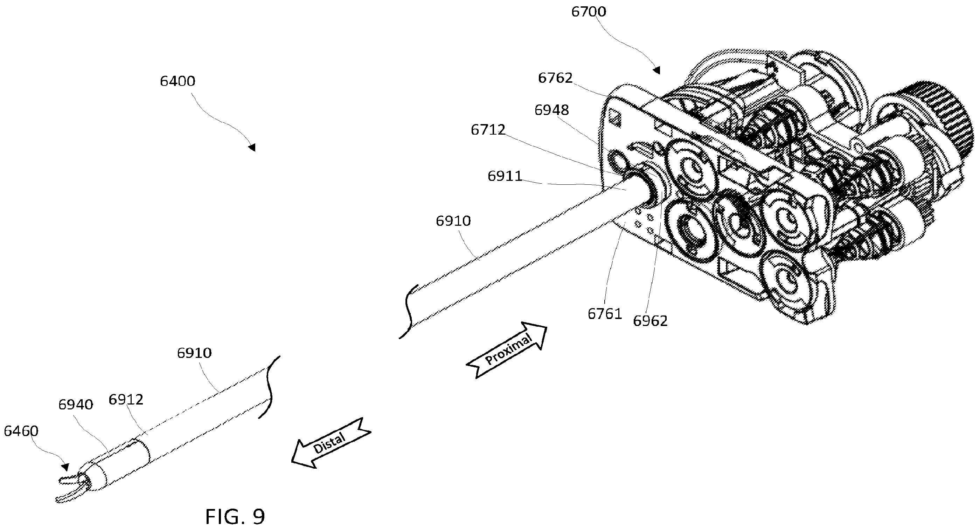

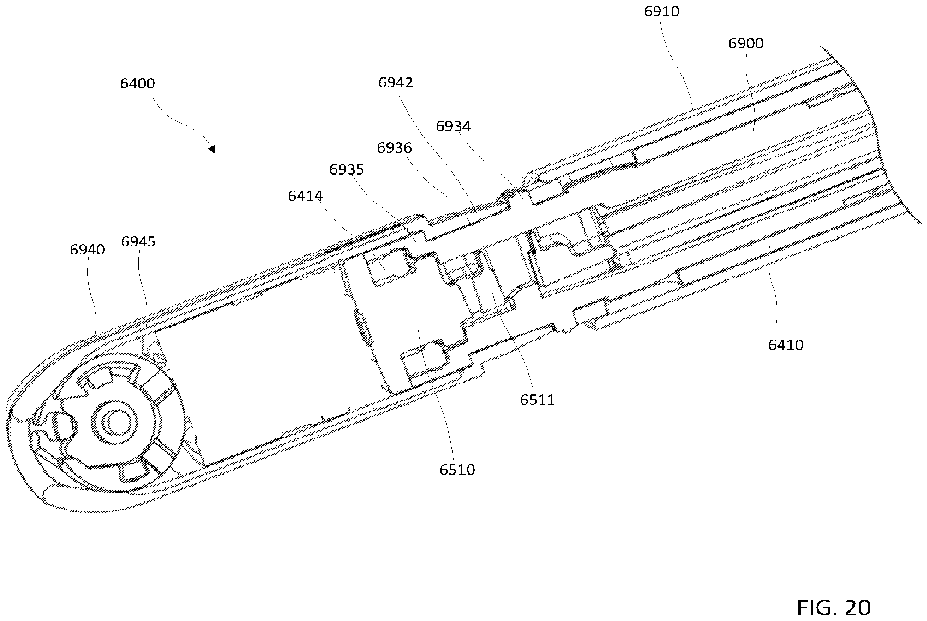

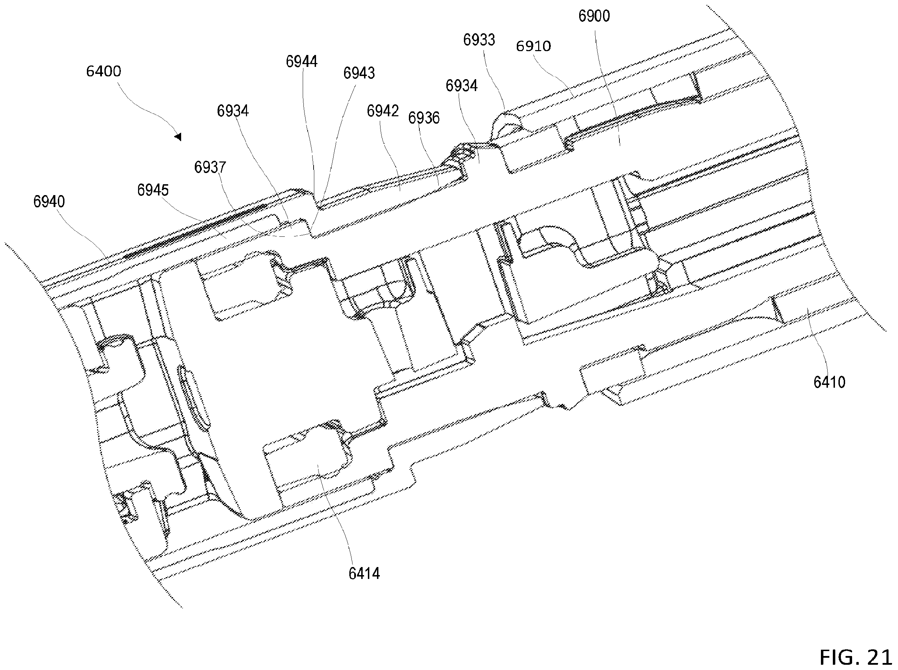

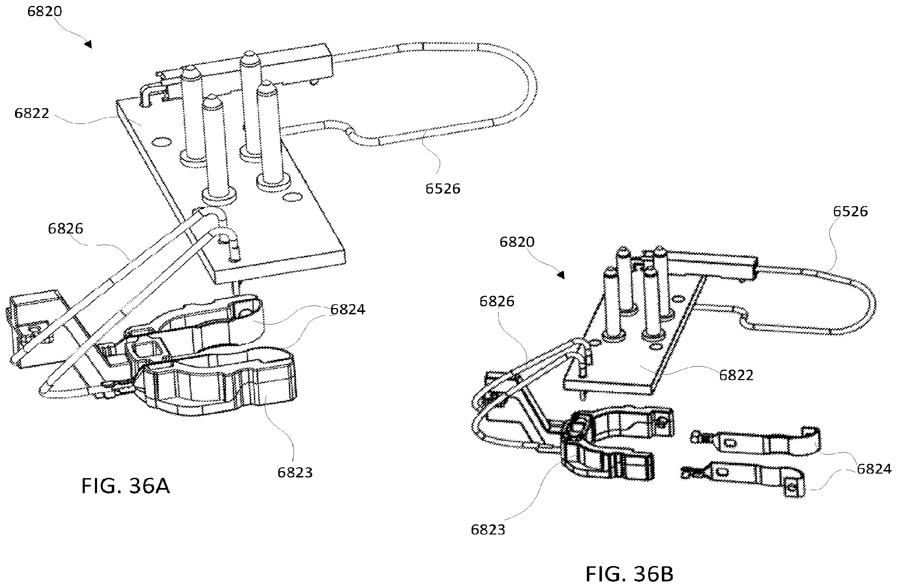

The end effector 2460 is rotatably coupled to the distal end portion 2412 of the shaft 2410 and includes at least one tool member 2462. The medical device 2400 is configured such that movement of the first proximal portion 2421 and the second proximal portion 2423 of the cable 2420 produces movement of the tool member 2462 about a first axis of rotation A1 (which functions as the yaw axis; the term *yaw* is arbitrary), in a direction of arrows AA₁. In some embodiments, the medical device 2400 can include a wrist assembly including one or more links 2510 that couples the end effector 2460 to the distal end portion 2412 of the shaft 2410. In such an embodiment, movement of the first proximal portion 2421 and the second proximal portion 2423 of the cable 2420 can also produce movement of the wrist assembly about a second axis of rotation (which functions as the pitch axis; the term *pitch* is arbitrary) or both movement of the wrist assembly and the end effector 2460. An embodiment with a wrist assembly is described herein with reference to FIGS. 9-16 and 19-38.

The tool member 2462 includes a contact portion 2464, configured to engage or manipulate a target tissue during a surgical procedure. For example, in some embodiments, the contact portion 2464 can include an engagement surface that functions as a gripper, cutter, tissue manipulator, or the like. In other embodiments, the contact portion 2464 can be an energized portion of the tool member that is used for cauterization or electrosurgical procedures. The end effector 2460 is operatively coupled to the mechanical structure 2700 such that the tool member 2462 rotates relative to shaft 2410 about the first axis of rotation A1 in the direction of the arrow AA₁. In this manner, the contact portion 2464 of the tool member 2462 can be actuated to engage or manipulate a target tissue during a surgical procedure. The tool member 2462 (or any of the tool members described herein) can be any suitable medical tool member. Moreover, although only one tool member 2462 is shown, in other embodiments, the medical device 2400 can include two or more moving tool members that cooperatively perform gripping or shearing functions.

The mechanical structure 2700 includes a chassis 2760, a first capstan 2710, and a second capstan 2720. The chassis 2760 provides the structural support for mounting or supporting and aligning the components of the mechanical structure 2700. For example, openings, protrusions, mounting brackets and the like can be defined in or on chassis 2760. In some embodiments, the chassis 2760 can include multiple portions, such as an upper chassis and a lower chassis. In some embodiments, a housing can optionally enclose at least a portion of the chassis 2760. The first capstan 2710 is mounted to the mechanical structure 2700 (e.g., within the housing 2760) via a first capstan support member (not shown). For example, the first capstan support member can be a mount, shaft, or any other suitable support structure to secure the first capstan 2710 to the mechanical structure 2700.

The second capstan 2720 is mounted to the mechanical structure 2700 (e.g., within the housing 2760) via a second capstan support member (not shown). For example, the second capstan support member can be a mount, shaft, or any other suitable support structure to secure the second capstan 2720 to the mechanical structure 2700. The first capstan 2710 can be operable to be rotated about an axis A3 in a direction DD, as shown in FIG. 5. The second capstan 2720 can be operable to be rotated about an axis A4 parallel to the axis A3.

The cable 2420 is routed from the mechanical structure 2700 to the end effector 2460 and then back to mechanical structure 2700, and the first end of the cable 2420 is coupled to the first capstan 2710 and the second end of the cable 2420 is coupled to the second capstan 2720 of the mechanical structure 2700. The cable 2420 extends from the first capstan 2710 along the shaft 2410, and the distal portion 2422 of the cable 2410 passes through the seal opening 2416 of seal 2414 and is coupled to the end effector 2460. Although the cable 2420 is shown extending within an interior passageway 2413 of the shaft 2410 in FIG. 5A, in other embodiments, the cable 2420 can be routed exterior to the shaft 2410. The cable 2420 extends from the end effector 2460 along or within the passageway 2413 of the shaft 2410 and the second end of the cable 2410 is coupled to the second capstan 2720 of the mechanical structure 2700. In other words, the two ends of a single cable (e.g., 2420) are coupled to and actuated by two separate capstans of the mechanical structure 2700.

More specifically, the two ends of the cable 2420 that are associated with opposing directions of a single degree of freedom are connected to two independent drive capstans 2710 and 2720. This arrangement, which is generally referred to as an antagonist drive system, allows for independent control of the movement of (e.g., pulling in or paying out) each of the ends of the cable 2420. The mechanical structure 2700 produces movement of the cable 2420, which operates to produce the desired articulation movements (pitch, yaw, or grip) at the end effector 2460. Accordingly, as described herein, the mechanical structure 2700 includes components and controls to move a first portion of the cable 2420 via the first capstan 2710 in a first direction (e.g., a proximal direction) and to move a second portion of the cable 2420 via the second capstan 2720 in a second opposite direction (e.g., a distal direction). The mechanical structure 2700 can also move both the first portion of the cable 2420 and the second portion of the cable 2420 in the same direction. In this manner, the mechanical structure 2700 can maintain the desired tension within the cables to produce the desired movements at the end effector 2460.

In other embodiments, however, any of the medical devices described herein can have the two ends of the cable wrapped about a single capstan. This alternative arrangement, which is generally referred to as a self-antagonist drive system, operates the two ends of the cable using a single drive motor.

In addition, in some alternative embodiments, the cable 2420 includes two cable segments, with each cable segment having a distal end portion that is coupled to the end effector 2460 and a proximal end portion wrapped about a capstan-either separate capstans as in the antagonist drive arrangement or a single common capstan in the self-antagonist drive arrangement. Descriptions herein referring to the use of a single cable 2420 incorporate the similar use of two separate cable segments.

With the cable 2420 coupled to the mechanical structure 2700 and to the end effector 2460, rotational movement produced by the first capstan 2710 causes the first proximal portion 2421 of the cable 2420 to move in a direction BB (e.g., proximally or distally depending on the direction of rotation), as shown in FIG. 5. Similarly, rotational movement produced by the second capstan 2720 causes the second proximal portion 2423 of the cable 2420 to move in the direction CC (e.g., proximally or distally depending on the direction of rotation), as shown in FIG. 5. Said another way, the first capstan 2710 can be operable to produce rotational movement about the axis A3, and the second capstan 2720 can similarly be operable to produce rotational movement about an axis A4 parallel to the axis A3. Thus, the first capstan 2710 can rotate in the direction of arrows DD and the second capstan 2720 can rotate in the direction of arrows EE in FIG. 5. For example, when the first capstan 2710 rotates about the axis A3 in a first direction (clockwise or counter-clockwise), the second capstan 2720 rotates about the axis A4 in either the same or the opposite direction (clockwise or counter-clockwise). Thus, as one of the capstans 2710, 2720 pays out the cable 2420, the other of the capstans 2710, 2720 pays in the cable 2420. Depending on how the cables are routed to the various capstans, it doesn't matter what direction each of the individual capstans rotates as long as the desired individual cable pay-in or pay-out is performed to perform the desired end effector motion-grip, yaw, or pitch-either alone or in combination.

With each of the ends of the cable 2420 coupled to a separate capstan, the movement of a first portion of the cable 2420 can be directly controlled by one capstan (e.g., first capstan 2710) and movement of a second portion of the cable 2420 can be directly controlled by the other capstan (e.g., second capstan 2720). Thus, the control of motion of the end effector 2460 in one direction is controlled by one capstan, and the control of motion of the end effector 2460 in the other direction is controlled by the other capstan. In this antagonist system, however, when the first capstan 2710 is controlling motion (i.e., applying tension to pull in the first proximal portion 2421 of the cable 2420), the second proximal portion 2423 of the cable is also under tension applied by the second capstan 2720. Maintaining tension applied by the non-driving capstan (i.e., the second capstan 2720) allows the non-driving capstan to immediately function as the driving capstan with no hysteresis in end effector control. The differing levels of tension applied by each capstan can also lead to improved control of the overall movement of the cable. Thus, better control of the overall movement of the end effector 2460 can be achieved. For example, accurate rotation in yaw around axis A1 can be controlled. The first capstan 2710 can be actuated to produce a rotational movement about the axis A3 in the direction of the arrow DD such that the first proximal portion 2421 of the cable is moved in a first direction along arrows BB. Simultaneously, the second capstan 2720 can be actuated to produce rotational movement about an axis parallel to the axis A3 in an opposite direction as the first capstan 2710 such that the second proximal end portion 2723 of the cable 2420 is moved in an opposite direction as the first proximal portion 2423 along arrows CC. Thus, the opposite movement of the first proximal portion 2421 and the second proximal portion 2423 causes the end effector 2460 to rotate (via the cable 2420 connection to the end effector 2460) about the rotational axis A1 (e.g., yaw movement).

In a similar way, accurate rotation in pitch around a second axis A2 (e.g., pitch; orthogonal to the yaw axis A1 described above) can be controlled. As described above, the first capstan 2710 can be actuated to produce a rotational movement about the axis A3 in the direction of the arrow DD, while simultaneously the second capstan 2720 can be actuated to produce rotational movement about the axis A4 parallel to the axis A3 in the direction of the arrow EE such that the first proximal portion 2421 of the cable and the second proximal portion 2423 of the cable 2420 are moved together in the same direction (along arrows BB and CC, respectively). The movement of the first proximal portion 2421 and the second proximal portion 2423 in the same direction causes the end effector 2460 (or a wrist mechanism) to rotate (via the cable 2420 connection to the end effector 2460) about a second rotation axis A2 in the direction of arrow AA₂ (e.g., pitch movement). Persons of skill in the art will understand that this action controls rotation around the second axis A2 in a first direction, and a similar action by an additional cable (or cable segments) (not shown) controls rotation around the second axis A2 in a second direction opposite the first direction. Thus, an antagonistic control relationship between the cable portions 2420 acting together and the additional cable is used to accurately control end effector rotation in pitch. Alternatively, a resiliency such as a spring may be used to act against cable portions 2420 to urge rotation around the second axis A2 in a direction opposite to the direction urged by cable portions 2420. Thus, the combination of the first capstan 2710, the second capstan 2720, and the single cable 2420 are operable to control the end effector 2460 of medical device 2400 in at least 2 DOFs (e.g., pitch and yaw).

The cable 2420, and any of the cables described herein can be made from any suitable materials. For example, in some embodiments, any of the cables described herein can be formed from an ultra-high molecular weight polyethylene (UHMWPE) fiber. In some embodiments, any of the cables described herein can be constructed from a single monofilament. In other embodiments, any of the cables described herein can be constructed from multiple cofilament strands, laid or woven (or both), or thermally fused, or otherwise combined to form the cable. In some embodiments, the cable 2420 or any of the cables described herein can include an optional outer sheath, coating, or other surface treatment to increase the frictional characteristics of the cable.

In some embodiments, the cable 2420 and any of the cables described herein can be made from a material having suitable temperature characteristics for use with cauterizing instruments. For example, such materials include liquid crystal polymer (LCP), aramid, para-aramid, and polybenzobisoxazole fiber (PBO). Such materials can provide frictional characteristics that increase the ability for friction coupling and improve holding ability, for example for coupling the cable 2420 to the capstan 2710 and end effector 2460. Such ability can also improve slip characteristics (e.g., help prevent the cable from slipping) during operation of the medical device. Such materials may or may not need a coating or other surface treatment to increase the frictional characteristic.

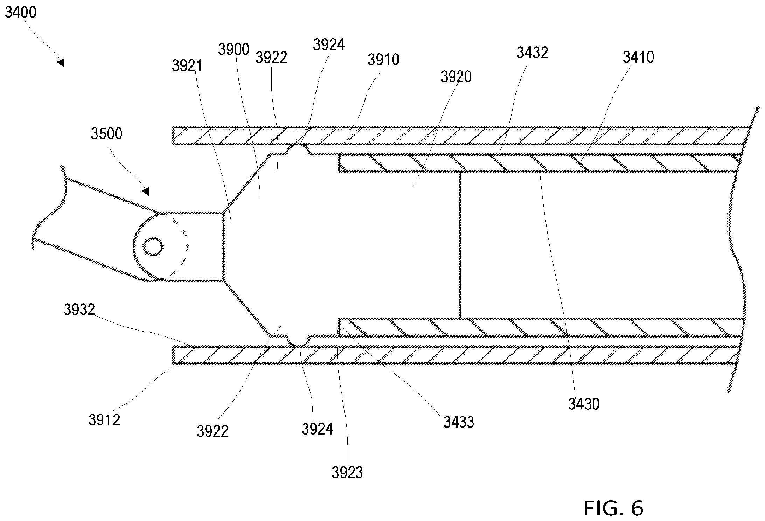

FIG. 6 is a schematic illustration of a distal end portion of a medical device 3400 according to an embodiment. In some embodiments, the medical device 3400 or any of the components therein are optionally parts of a surgical system that performs surgical procedures, and which can include a manipulator unit, a series of kinematic linkages, a series of cannulas, or the like. The medical device 3400 (and any of the instruments described herein) can be used in any suitable surgical system, such as the MIRS system 1000 shown and described above. The medical device 3400 includes a shaft 3410, a wrist assembly 3500, a spacer 3900 and an outer cover 3910. Although not shown, the medical device 3400 can also include other components of a medical device as a described herein, such as for example, a proximal mechanical structure, an end effector, a cable coupled between the mechanical structure and the end effector, etc. The shaft 3410 includes an inner surface 3430, an outer surface 3432 and a distal end surface 3433. The shaft 3410 can be formed, for example, with an electrically conductive material such as stainless steel.

The outer cover 3910 is positioned over the outer surface 3432 of the shaft 3410 and has an inner surface 3932 and a distal end portion 3912. The outer cover 3910 can be formed with, for example, an insulative material, such as, for example, Hytrel^{®} (TPC-ET thermoplastic polyester elastomer), TPU, Nylon^{®} material (Polyamide), ethylene tetrafluoroethylene (ETFE), polytetrafluoroethylene (PTFE), or other suitable material. The distal end portion 3912 extends distally beyond the out distal end surface 3433 of the shaft 3410. The spacer 3900 can be electrically insulative and can be formed with an insulative material, such as, for example, a polyphthalamide PPA GF material, or other suitable material. The spacer 3900 includes a first coupling portion 3920, a second coupling portion 3921, and a circumferential annular protrusion 3922. The first coupling portion 3920 is coupled to the inner surface 3430 of the shaft 3410 and the second coupling portion 3921 is coupled to the wrist assembly 3500. The annular protrusion 3922 includes a shoulder 3923 and a seal surface 3924. As shown in FIG. 6, the seal surface 3924 can be a surface of a smaller annular protrusion extending outwardly from the annular protrusion 3922. In other embodiments, the protrusion 3922 may not include such a smaller annular protrusion and instead the seal surface can be defined by the annular surface of the protrusion 3922.

The shoulder 3923 of the spacer 3900 contacts the distal end surface 3433 of the shaft 3410 and the shaft 3410 is isolated (electrically, fluidically, or both) by the seal surface 3924 of the annular protrusion 3922 contacting the inner surface 3932 of the outer cover 3910. For example, the medical device 3400 may include an end effector with a tool having blades that can be electrically charged to cut or cauterize tissue. The wrist assembly 3500 may include components formed in part with electrically conductive material to deliver energy to the tool. Thus, during a procedure using the medical device 3400, it is desirable to prevent or limit fluid contact with the electrically conductive shaft 3410 which acts as an energy shield. The shaft 3410 can become capacitively coupled and charged during the application of energy and the spacer 3900 and outer cover 3910 provide seal portions to isolate the shaft 3410, to prevent fluid contact with the shaft 3410 and to avoid unintended damage to tissue during treatment using the medical device 3400.

In some embodiments, the medical device 3400 can also include a seal as described herein, such as seal 2414 described above and seal 6414 described below. In such an embodiment, the spacer 3900 can define an internal passageway and the seal can be coupled within the passageway of the spacer 3900 at a distal end of the spacer 3900. Cables can extend between a proximal mechanical structure of the medical device 3400 and the wrist assembly, and pass through the passageway of the spacer 3900 and through cable seal openings of the seal. The seal can provide further protection to isolate the shaft 3410 from electrical energy and can assist in preventing liquids and/or gases from passing into the shaft 3410, as described herein.

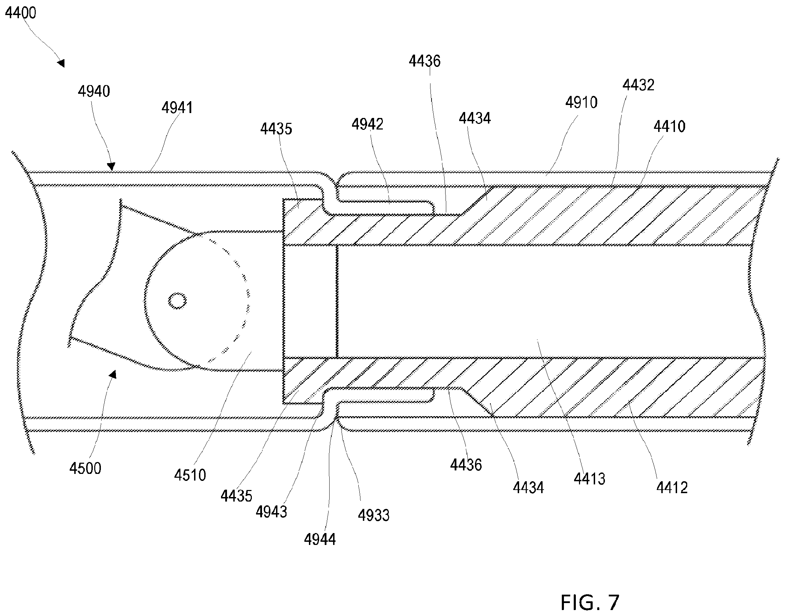

FIG. 7 is a schematic illustration of a distal end portion of a medical device 4400 according to an embodiment. In some embodiments, the medical device 4400 or any of the components therein are optionally parts of a surgical system that performs surgical procedures, and which can include a manipulator unit, a series of kinematic linkages, a series of cannulas, or the like. The medical device 4400 (and any of the instruments described herein) can be used in any suitable surgical system, such as the MIRS system 1000 shown and described above. The medical device 4400 includes a shaft 4410, a wrist assembly 4500, an outer cover 4910 and a distal tip cover 4940. Although not shown, the medical device 4400 can also include other components of a medical device as a described herein, such as for example, a proximal mechanical structure, an end effector, a cable coupled between the mechanical structure and the end effector, etc.

In this embodiment, the shaft 4410 includes a distal end portion 4412, an outer surface 4432, a first shoulder 4434, a second shoulder 4435 and a mounting recess 4436 between the first shoulder 4434 and the second shoulder 4435. The shaft 4410 defines a passageway 4413 that extends between a proximal end and a distal end of the shaft 4410. The shaft 4410 can be formed, at least in part with, for example, an electrically conductive material such as stainless steel. In some embodiments, the shaft can be constructed from multiple different materials or components. For example, the distal end portion 4412 can be formed with, for example, a polyphthalamide PPA GF material, or other suitable material. The remaining portion of the shaft 4410 can be formed with the same material as the distal end portion 4412 or with a different material. For example, the remaining portion of the shaft 4410 can be formed with a conductive material such as stainless steel. The outer cover 4910 can be formed with, for example, Hytrel^{®} material or one or more other insulative materials. The tip cover 4940 can be formed with, for example, silicone, or other suitable materials.

The wrist assembly 4500 is coupled to the distal end portion 4412 of the shaft 4410. For example, in some embodiments, a portion of the wrist assembly 4500 can be positioned within the passageway 4413 of the shaft 4410. The outer cover 4910 includes a distal end surface 4933 and surrounds the outer surface 4432 of the shaft 4410. The tip cover 4940 includes an outer surface 4941, a coupling protrusion 4942, a first seal surface 4943 and a second seal surface 4944. The tip cover 4940 at least partially surrounds the shaft 4410.

As shown in FIG. 7, the coupling protrusion 4942 of the tip cover 4940 is positioned within the mounting recess 4436 of the shaft 4410 between the outer cover 4910 and the shaft 4410 such that the first seal surface 4943 of the tip cover 4940 engages the second shoulder 4435 of the shaft 4410, and the second seal surface 4944 of the tip cover 4940 engages the distal end surface 4933 of the outer cover 4910. Thus, the first seal surface 4943 of the tip cover 4940 forms a first seal with the second shoulder 4435 of the shaft 4410, and the second seal surface 4944 of the tip cover 4940 forms a second seal with the distal end surface 4933 of the outer cover 4910. The tip cover 4940 and the outer cover 4910 prevent or limit the shaft 4410 from being exposed to liquids and/or insufflation gases during use of the medical device 4400 during a procedure. Said another way, this arrangement seals the fluid path F1 shown in FIG. 14D and described below. The tip cover 4940 and the outer cover 4910 also isolate the shaft 4410 when formed at least in part with a conductive material such as stainless steel, such that if the shaft 4410 becomes capacitively coupled and charged during a procedure, the shaft 4410 will not cause unintended damage to tissue.

In some embodiments, the medical device 4400 can also include a seal as described herein, such as seal 2414 described above and seal 6414 described below. In such an embodiment, the seal can be coupled within the passageway 4413 of the shaft 4410 at a distal end of the shaft 4410 and the wrist assembly 4500 can be coupled to the seal. Cables can extend between a proximal mechanical structure of the medical device 4400 and the wrist assembly 4500 and pass through the passageway of the shaft 4410 and through cable seal openings of the seal. The seal can provide further protection to isolate the shaft 4410 from electrical energy and can assist in preventing liquids and/or gases from passing into the shaft 4410, as described herein.

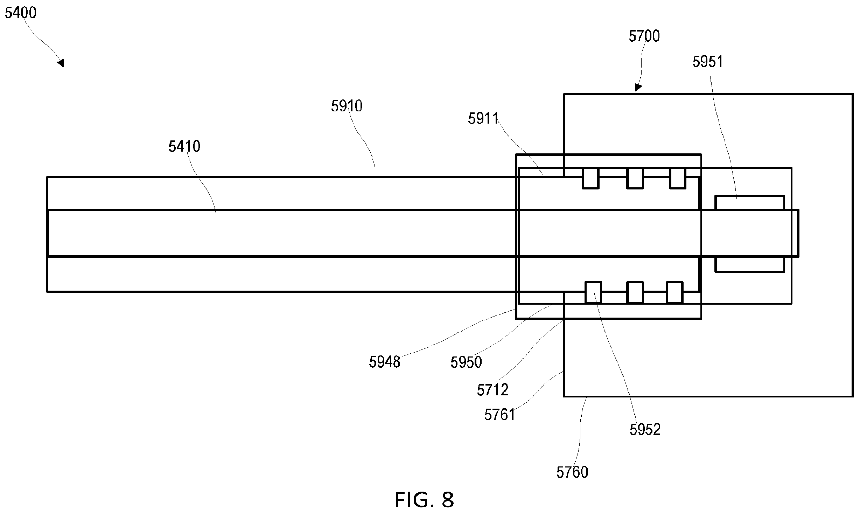

FIG. 8 is a schematic illustration of a proximal end portion of a medical device 5400 according to an embodiment. In some embodiments, the medical device 5400 or any of the components therein are optionally parts of a surgical system that performs surgical procedures, and which can include a manipulator unit, a series of kinematic linkages, a series of cannulas, or the like. The medical device 5400 (and any of the instruments described herein) can be used in any suitable surgical system, such as the MIRS system 1000 shown and described above. The medical device 5400 includes a shaft 5410, an outer cover 5910, a mechanical structure 5700, a flange 5950 and a lock ring 5948. Although not shown, the medical device 5400 can also include other components of a medical device as described herein for other embodiments, such as for example, an end effector, a cable coupled between the mechanical structure 5700 and the end effector, a tip cover, various components within the mechanical structure 5700 (e.g., capstans), etc.

The mechanical structure 5700 includes a chassis component 5760 having a distal end surface 5761. In some embodiments, the chassis component 5760 can include an upper chassis and a lower chassis that can partially enclose or fully enclose other components of mechanical structure 5700. In some embodiments, a housing cover (not shown) encloses the mechanical structure 5700, including the upper chassis component 5760. The chassis component 5760 provides structural support for mounting and aligning components in the mechanical structure 5700. For example, the chassis component 5760 includes a shaft opening 5712, within which a proximal end portion 5411 of the shaft 5410 is mounted.

The outer cover 5910 includes a proximal end portion 5911 that is coupled to the chassis component 5760 and extends into the opening 5712 of the chassis component 5760. The outer cover 5910 at least partially surrounds the shaft 5410. As with previous embodiments and other embodiments described herein, the shaft 5410 can be formed, for example, with an electrically conductive material such as stainless steel. The outer cover 5910 can be formed with, for example, an insulative material, such as, for example, TPU such that the outer cover 5910 can isolate the electrically conductive shaft 5410 as described herein.

The flange 5950 is coupled to the chassis component 5760 and is positioned such that a portion of the flange 5950 surrounds the shaft 5410 and a portion surrounds the outer cover 5910. The flange 5950 includes a first coupling portion 5951 and a second coupling portion 5952. The first coupling portion 5951 couples the flange 5950 to the shaft 5410 and the second coupling portion 5952 couples the flange 5950 to the outer cover 5910. In some embodiments, the first coupling portion 5951 includes multiple bosses that are received within openings defined in a wall of the shaft 5410. When the bosses are positioned within the openings of the shaft 5410, movement of the shaft 5410 relative to the flange 5950 in an axial direction is limited. The second coupling portion 5952 includes multiple locking teeth that engage the proximal end portion 5911 of the outer cover 5910. The lock ring 5948 extends at least partially within the opening 5712 of the chassis component 5760 and is positioned to urge the locking teeth to engage the proximal end portion 5911 of the outer cover 5910 to fixedly couple the outer cover 5910 to the shaft 5410. For example, the lock ring 5948 surrounds a portion of the flange 5950, which surrounds the proximal end portion 5911 of the outer cover 5910 such that the lock ring 5948 causes the locking teeth of the flange 5950 to press into the material of the outer cover 5910.

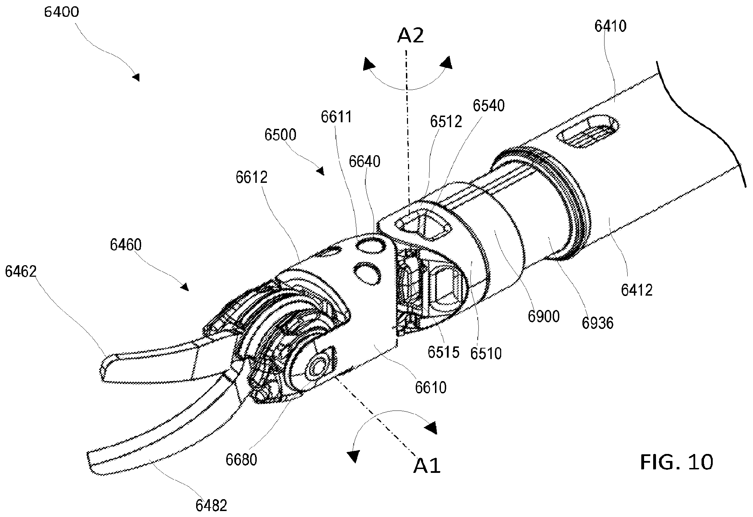

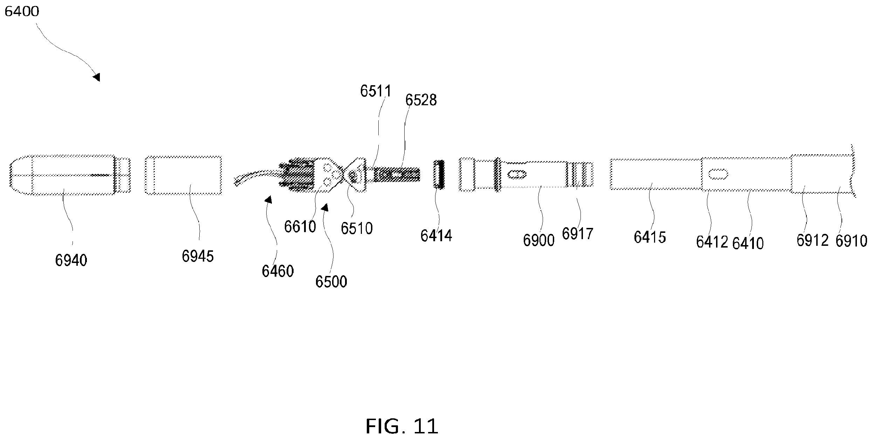

FIGS. 9-16 and 19-38 are various views of an instrument 6400, according to an embodiment. In some embodiments, the instrument 6400 or any of the components therein are optionally parts of a surgical system that performs surgical procedures, and which can include a manipulator unit, a series of kinematic linkages, a series of cannulas, or the like. The instrument 6400 (and any of the instruments described herein) can be used in any suitable surgical system, such as the MIRS system 1000 shown and described above. The instrument 6400 includes a proximal mechanical structure 6700, a shaft 6410 (see, e.g., FIGS. 10 and 11), an inner insulative cover 6415 (see, e.g., FIG. 11), an outer insulative cover 6910 (see e.g., FIG. 11), a distal wrist assembly 6500 (see FIGS. 10 and 11), a distal end effector 6460, a distal tip cover 6940, an inner tip cover 6945 (see, e.g., FIG. 11), a seal 6414 and a spacer 6900. Although not shown, the instrument 6400 also includes one or more cables that couple the proximal mechanical structure 6700 to the distal wrist assembly 6500 and end effector 6460. In some embodiments, the cables can be constructed from a polymer as described above for the cable 2420. The instrument 6400 is configured such that movement of one or more of the cables produces rotation of the end effector 6460 about a first axis of rotation A1 (see FIG. 10, which functions as a yaw axis, the term *yaw* is arbitrary), rotation of the wrist assembly 6500 about a second axis of rotation A2 (see FIG. 10, which functions as a pitch axis), a cutting rotation of the tool members of the end effector 6460 about the first axis of rotation A1, or any combination of these movements. Changing the pitch or yaw of the instrument 6400 can be performed by manipulating the cables in a similar manner as that described above for the medical device 2400.

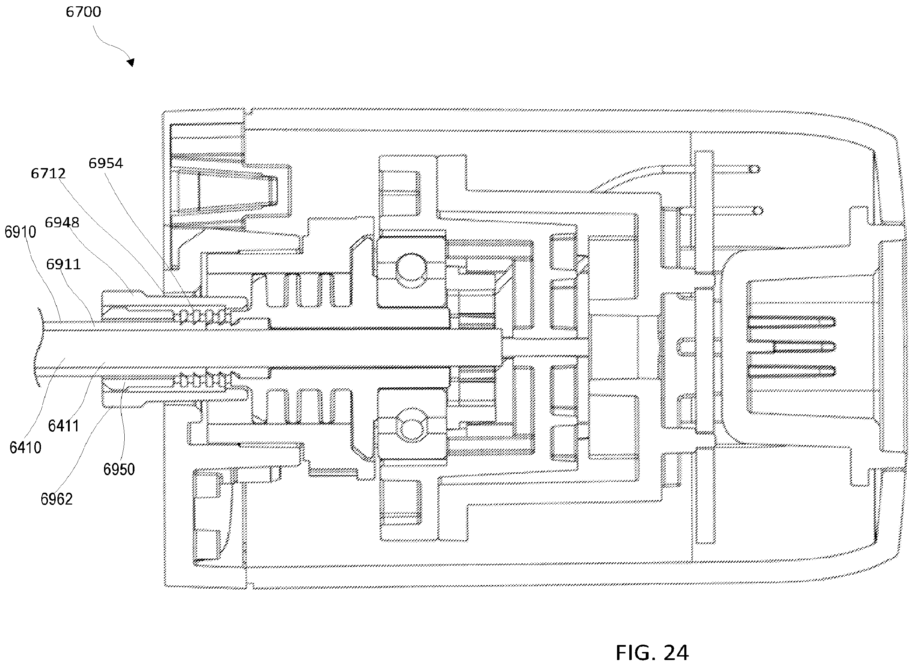

The shaft 6410 can be any suitable elongated shaft that is coupled to the wrist assembly 6500 and to the mechanical structure 6700. Specifically, the shaft 6410 includes a proximal end 6411 (see FIG. 24) that is coupled to the mechanical structure 6700, and a distal end 6412 that is coupled to the wrist assembly 6500 (e.g., a proximal link of the wrist assembly 6500). The inner insulative cover 6415 is disposed within a passageway of the shaft 6410. The shaft 6410 and the inner insulative cover 6415 define a passageway, through which the cables and other components (e.g., charged electrical wires, ground wires, or the like) can be routed from the mechanical structure 6700 to the wrist assembly 6500. The shaft 6410 can be formed, at least in part with, for example, an electrically conductive material such as stainless steel. The inner insulative cover 6415 can be formed with, for example, Hytrel^{®} material or one or more other insulative materials.

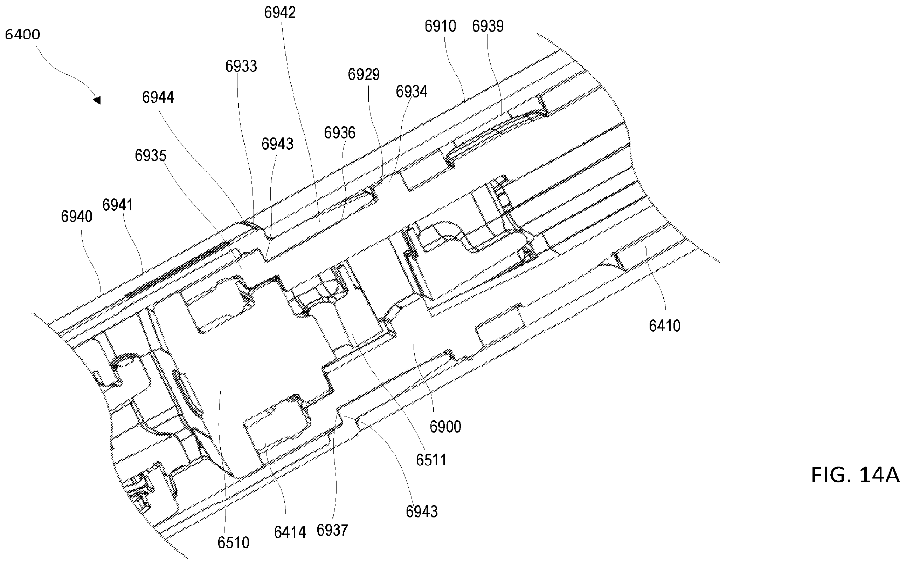

The outer cover 6910 can also be formed with, for example, Hytrel^{®} material or one or more other insulative materials. The outer cover 6910 includes a distal end surface 6933 and extends beyond a distal end of the shaft 6410 (see, e.g., FIG. 14A). The outer cover 6910 surrounds an outer surface of a portion of the spacer 6900 and an outer surface of at least a portion of the shaft 6410.

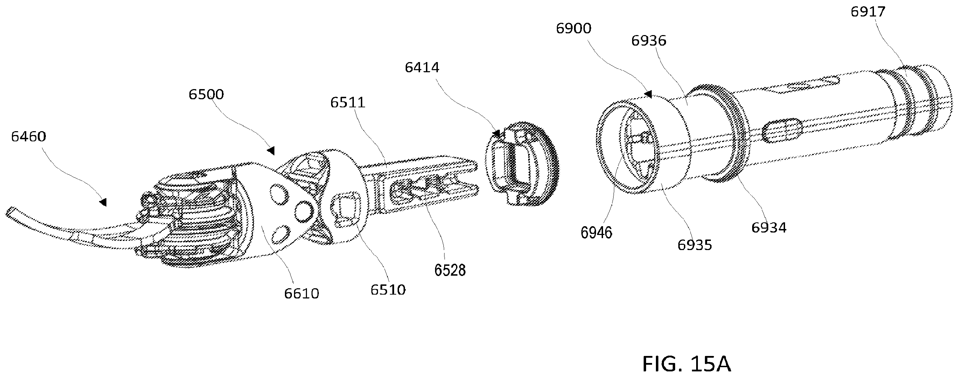

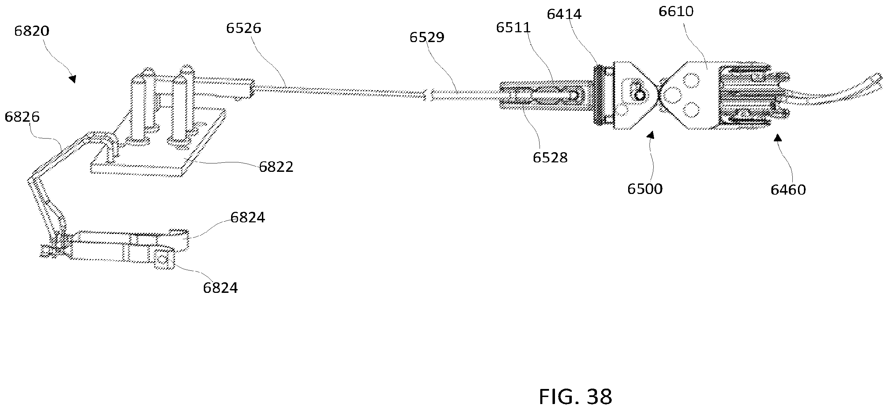

Referring to FIG. 10, the wrist assembly 6500 (also referred to as a joint assembly) includes a first link 6510, a second link 6610 and a third link 6515. The first link 6510 has a proximal portion 6511(see, FIGS. 11 and 15A) and a distal end portion 6512. The proximal portion 6511 includes a coupling protrusion that is coupled to the seal 6414 and the spacer 6900, as described in more detail below. The proximal portion 6511 can be coupled to the seal 6414 via any suitable mechanism. For example, in some embodiments, the proximal portion 6511 can be matingly disposed within a portion of the seal 6414 and spacer 6900 (e.g., via an interference fit). In some embodiments, the proximal portion 6511 can include one or more protrusions, recesses, openings, or connectors that couple the proximal portion 6511 to the seal 6414 and spacer 6900. In some embodiments, the proximal portion 6511 of the first link 6510 is coupled only to the seal 6414. In some embodiments, the proximal portion 6511 can be welded, glued, or fused to the seal 6414 and/or spacer 6900. At least some portions of the wrist assembly 6500 are formed with a metallic material and are used in the delivery of electrical energy to the tool members 6462 and 6482. For example, the first link 6510 and the second link 6610 can be formed with a metallic material. The coupling protrusion of the proximal portion 6511 of the link 6510 includes an interior region 6528 that can receive an electrical connector 6529 configured to receive an electrical wire 6526 to electrically couple the electrical wire 6526 to the proximal link 6511, as shown in FIG. 38 and as described in more detail below.

The distal end portion 6512 of the wrist assembly 6500 includes a joint portion 6540 that is rotatably coupled to a mating joint portion 6640 of the second link 6610 as described in more detail below. The second link 6610 has a proximal portion 6611 and a distal end portion 6612. The proximal portion 6611 includes a joint portion 6640 that is rotatably coupled to the joint portion 6540 of the first link 6510 to form the wrist assembly 6500 having the second axis of rotation A2 about which the second link 6610 rotates relative to the first link as shown in FIG. 10. The wrist assembly 6500 can include any suitable coupling mechanisms. In this embodiment, the first link 6510 is coupled to the third link 6515 via a pinned joint and the second link 6610 is coupled to the third link 6515 via a pinned joint. In this manner, the third link 6515 maintains the coupling between the first link 6510 and the second link 6610 during rotation of the second link 6610 relative to the first link 6510. Further, as described above, the distal end portion 6512 of the first link 6510 includes a joint portion 6540 that is rotatably coupled to a mating joint portion 6640 at the proximal end portion 6611 of the second link 6610.

As shown in FIG. 10, the end effector 6460 is coupled to the second link 6610. More specifically, the distal end portion 6612 of the second link 6610 includes a connector 6680 that is coupled to the end effector 6460 such that the end effector 6460 (e.g., tool members of the end effector) rotates relative to the wrist assembly 6500 about the first axis of rotation A1 (see, e.g., FIG. 10). The second axis of rotation A2 is non-parallel to the first axis of rotation A1. The first axis A1 also functions as a cutting axis as tool members rotate in opposition to each other as described in more detail below. Thus, the instrument 6400 provides at least three degrees of freedom (i.e., yaw motion about the first axis of rotation A₁, pitch rotation about the second axis of rotation A2, and a cutting motion about the first axis of rotation A1). In alternative embodiments, depending on the type of end effector, rather than a cutting motion, the end effector can provide motion for other actions, such as, for example, grasping, cauterizing, etc. about the first axis of rotation A1. The connector 6680 can be any suitable connector to rotatably couple the end effector 6460 to the wrist assembly 6500. For example, in some embodiments, the first link 6510 can include a clevis and a pin, such as the pinned joints shown and described in U.S. Patent No. US 9,204,923 B2 (filed Jul. 16, 2008), entitled "Medical Device Electronically Energized Using Drive Cables," which is incorporated herein by reference in its entirety.

As shown in FIGS. 9 and 10, the end effector 6460 includes a first tool member 6462 and a second tool member 6482. The first tool member 6462 and the second tool member 6482 each include a contact portion (not identified) configured to engage or manipulate a target tissue during a surgical procedure. In this embodiment, the contact portion of the first tool member 6462 and the second tool member 6482 include an engagement surface that functions as a cutter (e.g., a cutting blade). The cutting blades can be any suitable blades, such as those shown and described in copending U.S. Provisional Patent Application Serial No. 63/234,662, entitled "Surgical Instrument Shears," the disclosure of which is incorporated herein by reference in its entirety. The first tool member 6462 and the second tool member 6482 can also be energized to use for cauterization or electrosurgical procedures. The end effector 6460 can be operatively coupled to the mechanical structure 6700 such that the tool members 6462 and 6482 rotate about the first axis of rotation A1. In this manner, the contact portions of the tool member 6462 and the contact portion of the tool member 6482 can be actuated to engage or manipulate a target tissue during a surgical procedure.

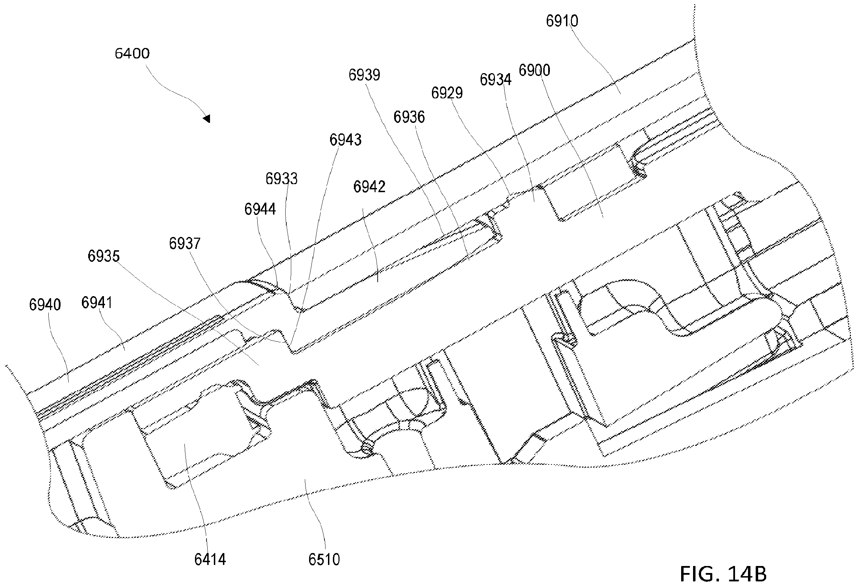

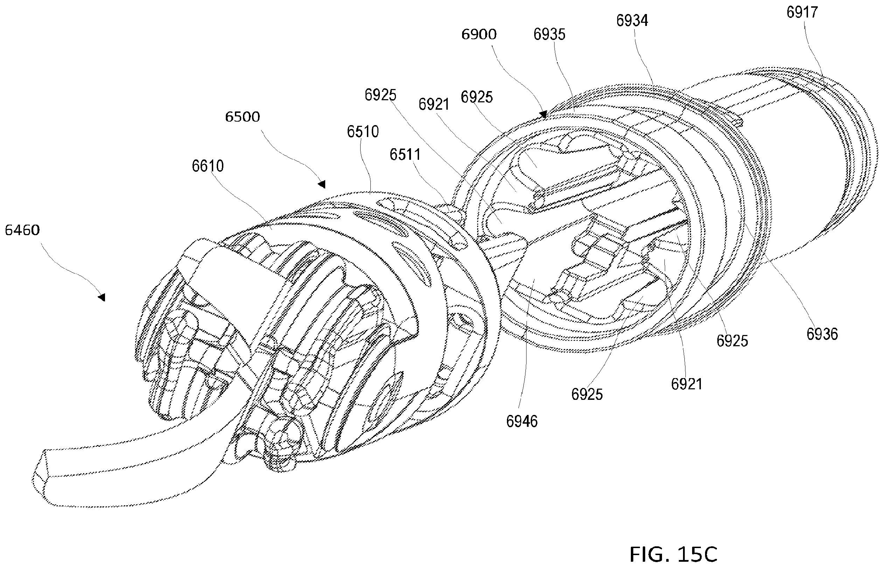

The spacer 6900 includes a first shoulder 6934, a second shoulder 6935, a proximal coupling portion 6917 (see, e.g., FIGS. 11 and 15A), and a mounting recess 6936 between the first shoulder 6934 and the second shoulder 6935. As shown, for example, in FIGS. 14A and 14B, the first shoulder 6934 of the spacer 6900 has a seal surface 6929 that engages an inner surface 6939 of the outer cover 6910, and the second shoulder 6935 has a seal surface 6937 that engages a seal surface 6943 of the distal tip cover 6940 described in more detail below. The first shoulder 6934 includes a barb 6929 that engages the outer cover 6910 and prevents the outer cover 6910 from moving proximally. The proximal coupling portion 6917 is received within a distal end of the inner insulative cover 6415. Said another way, the inner insulative cover 6415 is coupled about the proximal coupling portion 6917. The spacer 6900 includes an interior passageway 6946, and inner coupling portions 6921 (see, e.g., FIG. 15C) that define openings 6925 that can receive cables therethrough as described in more detail below. The coupling portions 6921 engage the protrusion of the proximal portion 6511 of the first link 6510. The spacer 6900 can be electrically insulative and formed with, for example, a polyphthalamide PPA GF material, or other suitable material.

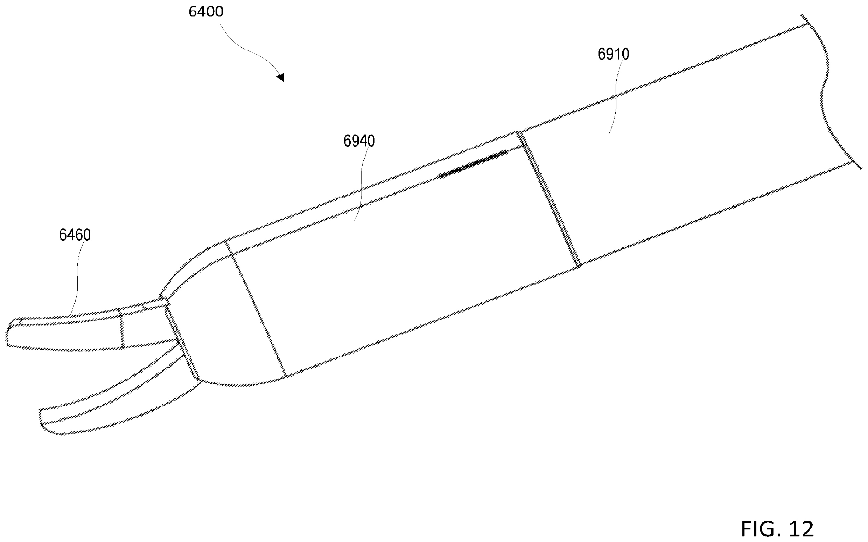

As shown in FIGS. 12-14B, the distal tip cover 6940 and inner tip cover 6945 are positioned over the wrist assembly 6500 and a portion of the end effector 6460. The distal tip cover 6940 can be formed with, for example, silicone or other suitable materials. The inner tip cover 6945 is coupled to an interior surface of the distal tip cover 6940 and can be formed with, for example, TPU or other suitable material. The distal tip cover 6940 can protect tissue in the treatment area from electrically charged wrist assembly components, and the inner tip cover 6945 can protect the distal tip cover 6940 from damage caused by movement of the cutting shears.

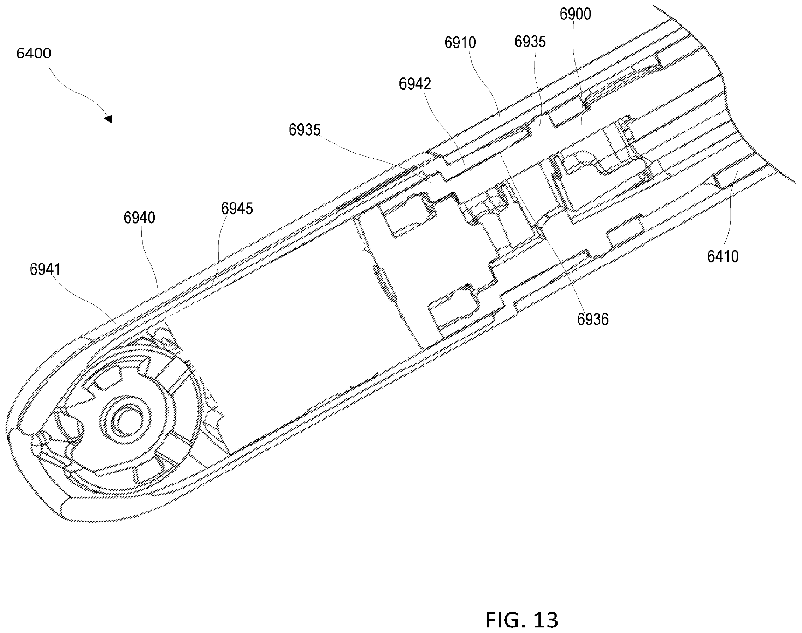

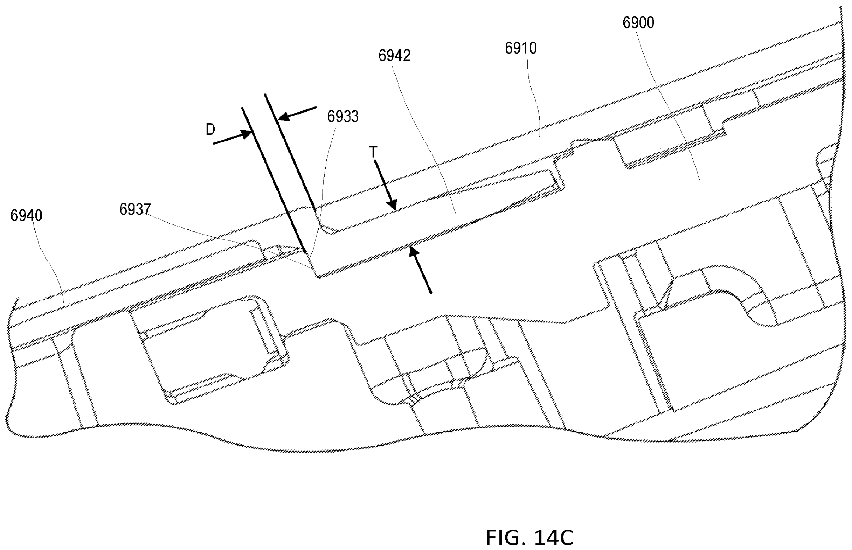

The distal tip cover 6940 includes an outer surface 6941, a coupling protrusion 6942, a first seal surface 6943, and a second seal surface 6944. As best shown in FIGS. 14A and 14B, the coupling protrusion 6942 of the distal tip cover 6940 is positioned within the mounting recess 6936 of the spacer 6900 between the outer cover 6910 and the spacer 6900 such that the first seal surface 6943 of the distal tip cover 6940 engages the seal surface 6937 of the first shoulder 6935 of the spacer 6900, and the second seal surface 6944 of the distal tip cover 6940 engages a distal end surface 6933 of the outer cover 6910. Thus, the first seal surface 6943 of the distal tip cover 6940 forms a first seal with the first shoulder 6935 of the spacer 6900, and the second seal surface 6944 of the distal tip cover 6940 forms a second seal with the distal end surface 6933 of the outer cover 6910. Further, the first shoulder 6934 of the spacer 6900 forms a seal with the inner surface 6939 of the outer cover 6910. As described above for previous embodiments, the distal tip cover 6940 and the outer cover 6910 prevent or limit the shaft 6410 from being exposed to liquids and/or insufflation gases during use of the medical device 6400 during a procedure. The distal tip cover 6940 and the outer cover 6910 also isolate the electrically conductive shaft 6410 such that if the shaft 6410 becomes capacitively coupled and charged during a procedure, the shaft 6410 will not cause unintended damage to tissue. As shown, in FIGS. 13-14D, when the coupling protrusion 6942 of the distal tip cover 6940 is positioned within the mounting recess 6936 of the spacer 6900 between the outer cover 6910 and the spacer 6900, and the outer cover 6910 is in the advanced position, the coupling protrusion 6942 of the distal tip cover 6940 is trapped between the outer cover 6910 and the spacer 6900. As shown in FIG. 14C, a maximum thickness T of the coupling protrusion 6942 is greater than a distance D between the end surface 6933 of the outer cover 6910 and the seal surface 6937 of the spacer 6900 such that the coupling protrusion 6942 cannot be removed when the outer cover 6910 is in the advanced position.

FIG. 14D illustrates an example flow paths F1 and F2 through which fluids (i.e., liquids and/or gases) can potentially travel, and which are sealed by the arrangement of various interactions between the tip cover 6940, the outer cover 6910, the seal 6414, and the spacer 6900, as described herein. Flow path F1 illustrates a potential flow along an inner surface of the distal tip cover 6940 and the inner tip cover 6945, between the distal tip cover 6940 and the spacer 6900, and between distal tip cover 6940 and the outer cover 6910. The leak path F2 illustrates a potential flow path through cable openings of the seal 6414, discussed in more detail below.

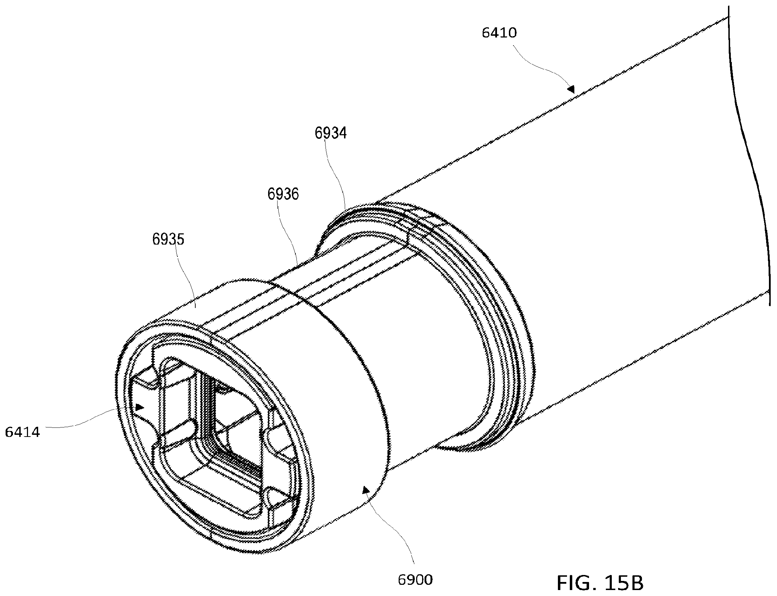

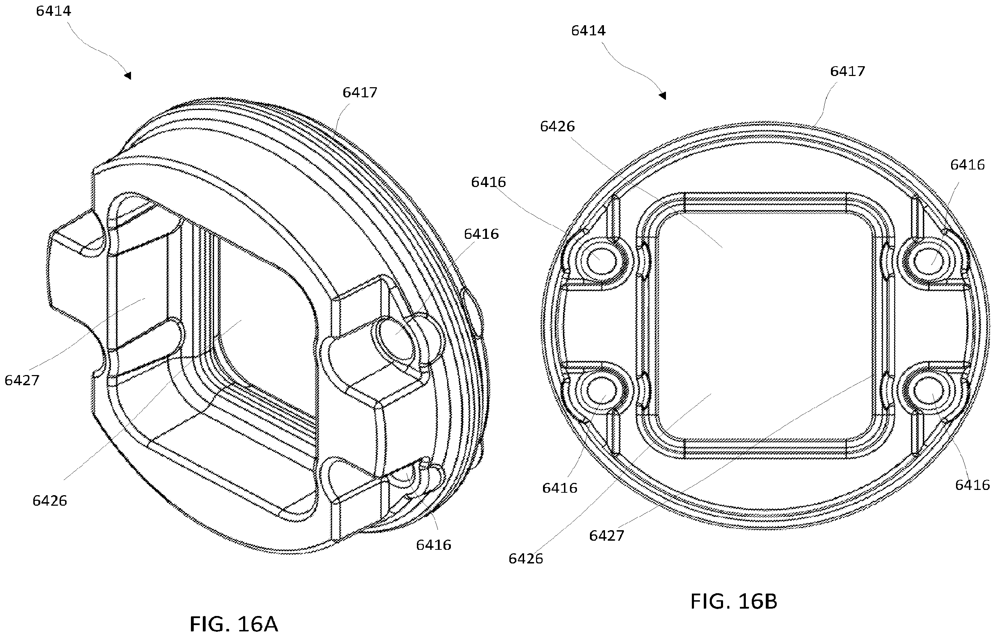

FIG. 15A is exploded view illustrating select components of the distal end portion of the medical device 6400, FIG. 15B is a perspective distal view of the seal 6414 and spacer 6900, and FIG. 15C is a partial exploded view illustrating a distal end of the spacer 6900 without the seal 6414 positioned within the spacer 6900. As shown in FIG. 15B, in this embodiment, the seal 6414 is positioned within the passageway 6946 of the spacer 6900 (see, e.g., FIGS. 15A and 15C). The protrusion of the proximal portion 6511 of the first link 6510 is received through the center opening 6426 (see, e.g., FIGS. 16A-16C) of the seal 6414 and passes through the center opening 6426 and within the passageway 6946 of the spacer 6900. The coupling portions 6921 (see, e.g., FIG. 15C) of the spacer 6900 provide a seal between the proximal portion 6911 of the link 6510 and the spacer 6900.

The seal 6414 can be formed with, for example, *Santoprene*^{™} thermoplastic vulcanizates (TPVs), a thermoplastic polyurethane (TPU) material, or other suitable material, and be coupled to an inner surface of the spacer 6900 by a friction fit, adhesive or other suitable coupling methods. As shown in FIGS. 16A-16D, the seal 6414 includes four cable seal openings 6416 through which a cable (not shown), such as cable 2420 described above can pass through. More specifically, the cable (e.g., cable 2420) can include a first end, a second end, a first proximal portion, a second proximal portion and a distal portion. The first end and the second end are each coupled to the mechanical structure 6700, the first proximal portion and the second proximal portion can extend through a passageway of the shaft 6410, and the distal portion extends through openings 6925 (see, e.g., FIG. 15C) of the spacer 6900, and through the cable seal openings 6416 and are coupled to the end effector 6460 as described above for medical device 2400.

As shown, for example, in FIGS. 16A and 16B, the seal 6414 defines an outer seal portion 6417 and an inner seal portion 6427 to limit fluid leakage into the spacer 6900 and shaft 6410, and also limit leakage of insufflation gas out the interior body cavity of the patient being treated via the medical device 6400. As described above, such leakage of liquid and/or insufflation gas into the shaft 6410 can be undesirable, for example, when the medical device 6400 includes electrically charged instruments. The outer seal portion 6417 provides a seal between an inner surface of the spacer 6900 and an outer perimeter of the seal 6414, and the inner seal portion 6427 provides a seal between an outer surface of the proximal portion 6511 of the link 6510 and an inner surface of the seal 6414.

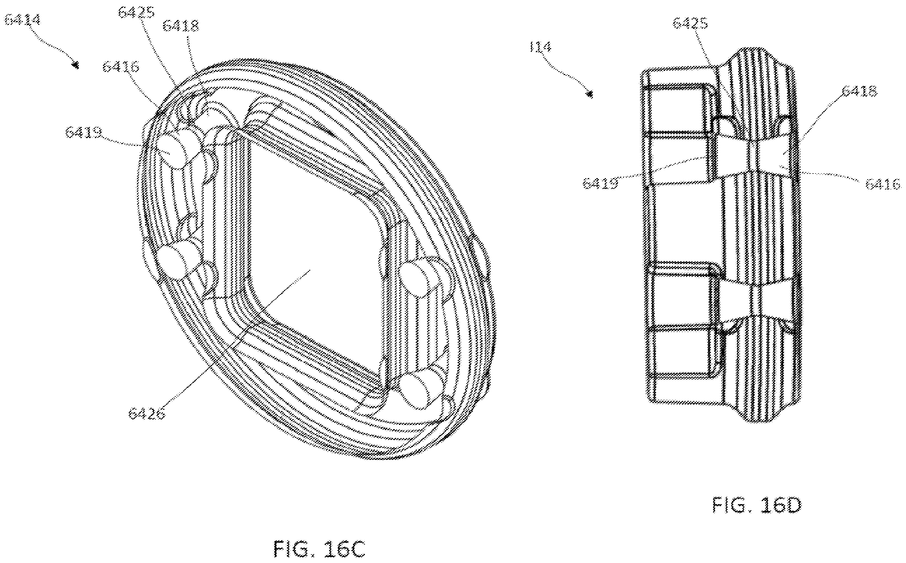

The cable seal openings 6416 are shaped and sized to also limit liquid and/or insufflation gas from leaking through the cable seal openings 6416. As described above with reference to FIG. 14C, a potential flow path F2 is through the cable seal openings 6416. More specifically, as shown in FIGS. 16C and 16D, the cable seal openings 6416 have an hourglass shape that has a first tapered portion 6418 at a first end of the openings 6416, a second tapered portion 6419 at a second end, and a throat portion 6425 having a smaller diameter than a diameter of the first tapered portion 6418 and a diameter of the second tapered portion 6419. The hourglass shape of the cable seal openings 6416 and the diameter of the throat portion 6425 provide for a tight fit to the cables that deforms the cables under tension to prevent liquid and/or gas leakage through the openings 6416. For example, the cables can be formed with a polymeric material such that the cable can deform under tension (as described above for cable 2420 and FIGS. 5B-5D). As described above, the cables can have a first nominal or untensioned state that has a first diameter, and a second, tensioned state with a second diameter. The first diameter of the cables is larger than the diameter of the cable seal openings 6416, and the second diameter of the cables is smaller than the diameter of the cable seal openings 6416. Thus, the cables can be passed through the cable seal openings 6416 when in the tensioned state and when the cable is untensioned, the cable will return to its untensioned, nominal first diameter and form a tight fit to the throat portion 6425 of the cable seal openings 6416. The cable seal openings 6416 are configured to protect against escaping insufflation pressure and minimize fluid leakage around the cables of the medical device 6400 without causing excessive friction when the cables are moved through the cable seal openings 6416.

As described above for cables 2420, in some embodiments, the cables used in medical device 6400 can be a polymeric braided construction. In some embodiments, a distal end portion of the cables and/or a portion of the seal 6414 that defines the cable seal openings 6416 can include an oil coating. In some embodiments, a distal end portion of the cables and/or a portion of the seal 6414 that defines the cable seal openings 6416 can include a hydrophobic material.

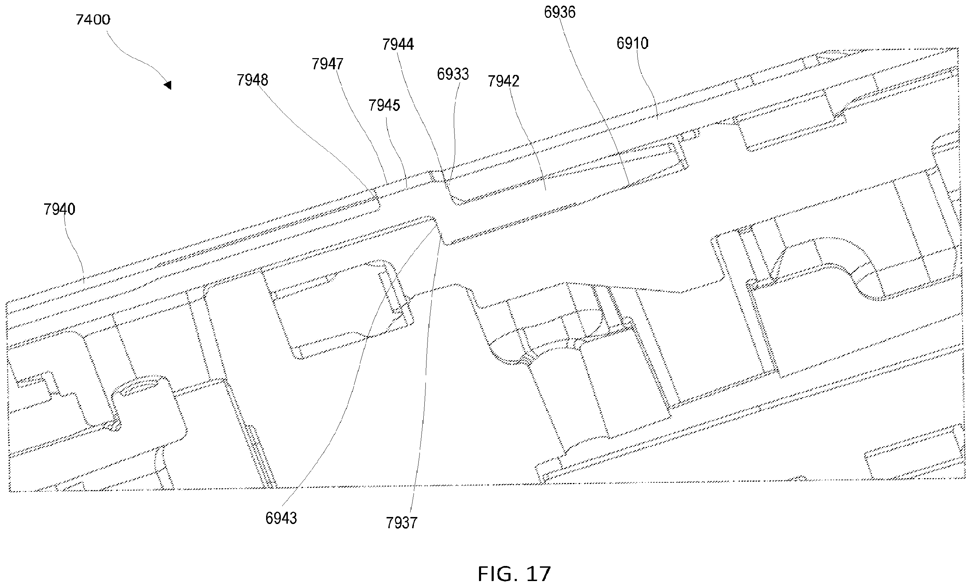

FIG. 17 illustrates an alternative embodiment of the distal tip cover and inner tip cover. An inner tip cover 7945 can be coupled to a distal tip cover 7940. In this embodiment, the inner tip cover 7945 includes a stepped portion 7947 that engages a proximal end 7948 of the distal tip cover 7940. The inner tip cover 7945 includes a coupling protrusion 7942, a first seal surface 7943, and a second seal surface 7944. The coupling protrusion 7942 of the inner tip cover 7945 is positioned within the mounting recess 6936 of the spacer 6900 between the outer cover 6910 and the spacer 6900 such that the first seal surface 7943 of the inner tip cover 7945 engages the seal surface 6937 of the first shoulder 6935 of the spacer 6900, and the second seal surface 7944 of the inner tip cover 7945 engages the distal end surface 6933 of the outer cover 6910. Thus, the first seal surface 7943 of the inner tip cover 7945 forms a first seal with the first shoulder 6935 of the spacer 6900, and the second seal surface 7944 of the inner tip cover 7945 forms a second seal with the distal end surface 6933 of the outer cover 6910. In this embodiment, the inner tip cover 7945 and the outer cover 6910 prevent or limit the shaft 6410 from being exposed to liquids and/or insufflation gases during use of the medical device 6400 during a procedure. Said another way, this arrangement seals the fluid path F1 shown in FIG. 14D. The inner tip cover 7945 and the outer cover 6910 also isolate the electrically conductive shaft 6410 such that if the shaft 6410 becomes capacitively coupled and charged during a procedure, the shaft 6410 will not cause unintended damage to tissue.

Similar to the previous embodiment, the distal tip cover 7940 and inner tip cover 7945 are positioned over the wrist assembly 6500 and a portion of the end effector 6460. The distal tip cover 7940 can be formed with, for example, silicone or other suitable materials. The inner tip cover 7945 can be formed with, for example, TPU or other suitable material. The distal tip cover 7940 and the inner tip cover 7945 can protect tissue in the treatment area from electrically charged wrist assembly components, and the inner tip cover 7945 can protect the distal tip cover 7940 from damage.

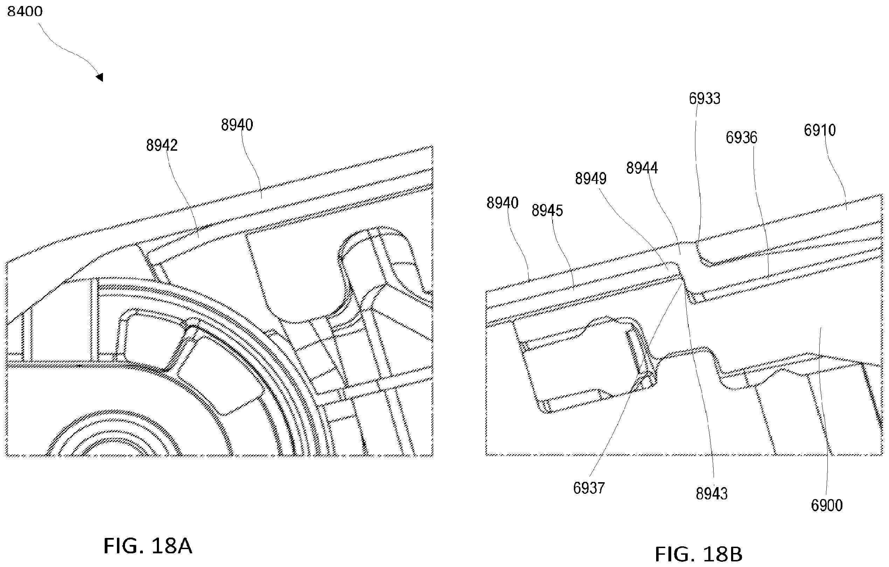

FIGS. 18A and 18B illustrate yet another alternative embodiment for a distal tip cover 8940 and an inner tip cover 8945. FIG. 18A illustrates a distal end of an inner tip cover 8945, and FIG. 18B illustrates a proximal end of the inner tip cover 8945. In this embodiment, the inner tip cover is not bonded to an inner surface of the distal tip cover 8940. A proximal end 8949 of the inner tip cover 8945 extends to a first seal surface 8943 of the distal tip cover 8945 (FIG. 18B) and a distal end 8951 of the inner tip cover 8945 extends within the distal tip cover 8940 to a distal portion of the distal tip cover 8940. The distal tip cover 8940 includes a coupling protrusion 8942, a first seal surface 8943, and a second seal surface 8944. The coupling protrusion 8942 of the distal tip cover 8940 is positioned within the mounting recess 6936 of the spacer 6900 between the outer cover 6910 and the spacer 6900 such that the first seal surface 8943 of the distal tip cover 8940 engages the seal surface 6937 of the first shoulder 6935 of the spacer 6900, and the second seal surface 8944 of the distal tip cover 8940 engages the distal end surface 6933 of the outer cover 6910. Thus, the first seal surface 8943 of the distal tip cover 8940 forms a first seal with the first shoulder 6935 of the spacer 6900, and the second seal surface 8944 of the distal tip cover 6940 forms a second seal with the distal end surface 6933 of the outer cover 6910.

As described above for previous embodiments, the distal tip cover 8940 and the outer cover 8910 can prevent or limit the shaft 6410 from being exposed to liquids and/or insufflation gases during use of the medical device 6400 during a procedure. The distal tip cover 8940 and the outer cover 6910 also isolate the electrically conductive shaft 6410 such that if the shaft 6410 becomes capacitively coupled and charged during a procedure, the shaft 6410 will not cause unintended damage to tissue.

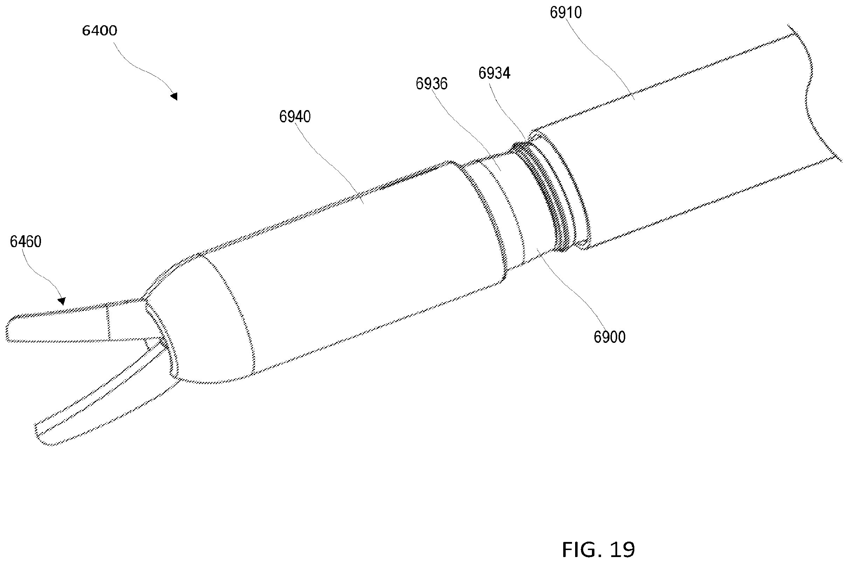

In some embodiments, the outer cover 6910 can be moved during the assembly of the medical device 6400 to facilitate assembling the tip cover. As shown, for example, in FIGS. 13-14D, when the medical device 6400 is assembled, the coupling protrusion 6942 of the distal tip cover 6940 is trapped between the outer cover 6910 and the spacer 6900. To assemble the medical device 6400, and position the coupling protrusion 6942 within the mounting recess 6936 of the spacer 6900, the outer cover 6910 is moved to a retracted position as shown in FIGS. 19-21, in which the outer cover 6910 is moved proximally such that the distal end 6933 of the outer cover 6910 is spaced proximally of the mounting recess 6936 of the spacer 6900. After the distal tip cover 6940 (and inner tip cover 6945 coupled thereto) are positioned within the mounting recess 6936, the outer cover 6910 can be moved to an advanced (i.e., distal) position as shown in FIGS. 12-14C. The proximal mechanical structure 6700 provides for mechanisms to allow for moving the outer cover 6910 between the retracted and advanced positions and for locking the outer cover 6910 in the advanced position, as described in more detail below.

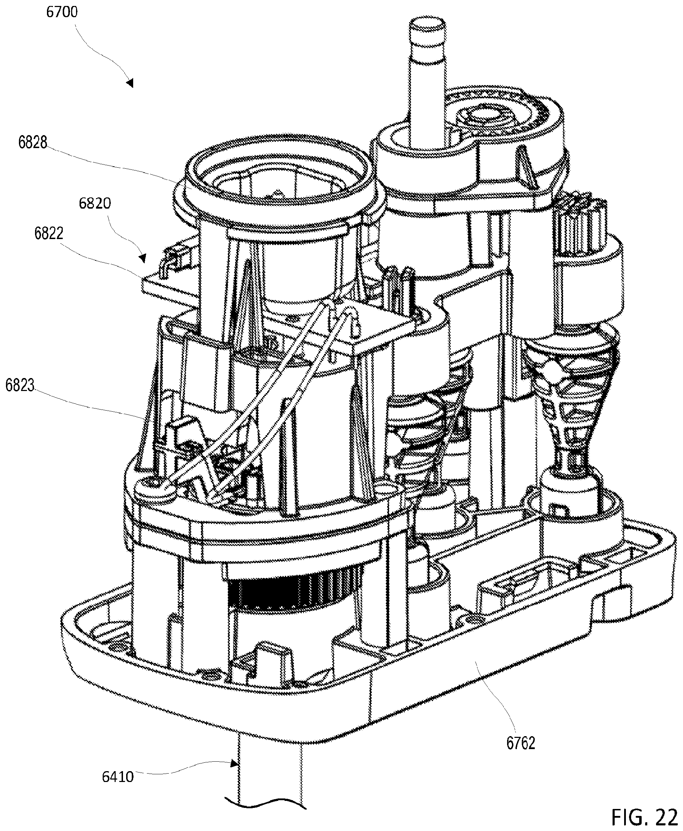

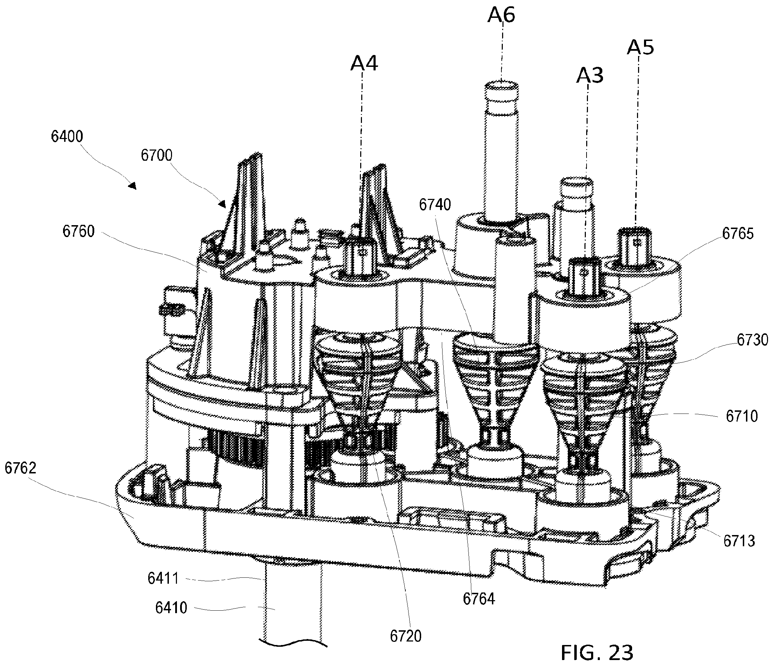

FIGS. 22 and 23 illustrate the proximal mechanical structure 6700. The mechanical structure 6700 includes an upper chassis 6760, a lower chassis 6762, four capstans 6710, 6720, 6730, 6740, and a cable guide 2800. In some embodiments, the upper chassis 6760 and the lower chassis 6762 may partially enclose or fully enclose other components of mechanical structure 6700. In some embodiments, a housing cover (not shown) encloses the mechanical structure 6700, including the upper chassis 6760 and the lower chassis 6762. The lower chassis 6762 and the upper chassis 6760 provide structural support for mounting and aligning components in the mechanical structure 6700. For example, the lower chassis 6762 includes a shaft opening 6712 (see FIG. 9), within which the proximal end 6411 of the shaft 6410 is mounted. The lower chassis 6762 further includes one or more bearing surfaces or openings 6713, within which the capstans 6710, 6720, 6730, and 6740 are mounted and rotatably supported. The upper chassis 6760 also includes openings (not shown) in a bottom 6764, within which an upper portion of the capstans are mounted. The openings of the upper chassis 6760 are axially aligned with the openings 6713 of the lower chassis 6762 to support the capstans. In addition to providing mounting support for the internal components of the mechanical structure 6700, the lower chassis 6762 can include external features (e.g., recesses, clips, etc.) that interface with a docking port of a drive device (not shown). The drive device can be, for example, a handheld system or a computer-assisted teleoperated system that can receive the instrument 6400 and manipulate the instrument 6400 to perform various surgical operations. The drive device can include one or more motors to drive capstans of the mechanical structure 6700. In other embodiments, the drive device can be an assembly that can receive and manipulate the instrument 6400 to perform various operations.

Each of capstans 6710, 6720, 6730, 6740 can be driven by a corresponding motor (not shown) in the drive device. For example, as shown in FIG. 23, the first capstan 6710 can be driven to rotate about a first capstan axis A3, the second capstan 6720 can be driven to rotate about a second capstan axis A4, the third capstan 6730 can be driven to rotate about a third capstan axis A5, and the fourth capstan 6740 can be driven to rotate about a fourth capstan axis A6.

As described above, during assembly of the medical device during manufacturing, the outer cover 6910 can be moved between a retracted position to enable the coupling protrusion 6942 of the distal tip cover 6940 to be positioned within the mounting recess 6936 of the spacer 6900, and an advanced position in which the outer cover 6910 can be locked in position and the coupling protrusion 6942 will be trapped between the outer cover 6910 and the spacer 6900. The proximal mechanical structure 6700 provides mechanisms to enable the movement of the outer cover 6910 and for locking the outer cover 6910 in the advanced position for use of the medical device 6400.

FIG. 24 is a cross-sectional view of the mechanical structure 6700, a proximal end portion 6911 of the outer cover 6910, a proximal end portion 6411 of the shaft 6410, and a proximal end portion of the inner insulative cover 6415. FIG. 24 illustrates the outer cover 6910 locked in the advanced (i.e., distal) position. As shown, for example, in FIGS. 24-26A, the proximal end portion 6911 of the outer cover 6910 and the proximal end portion 6411 of the shaft 6410 extend within the opening 6712 of the lower chassis 6762.

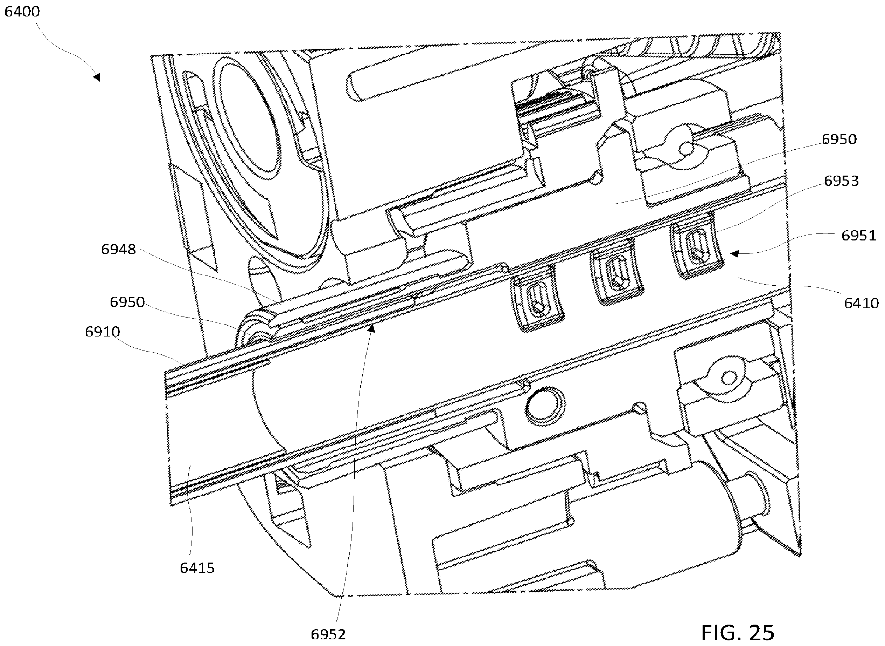

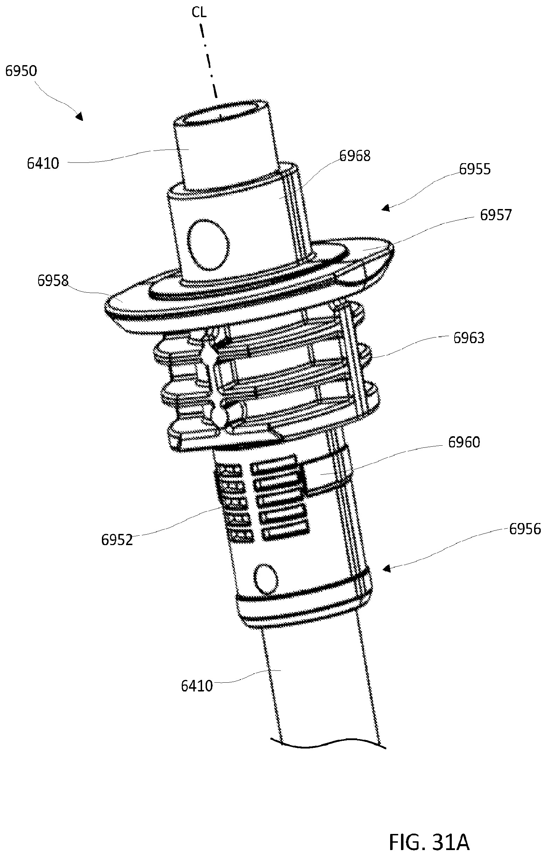

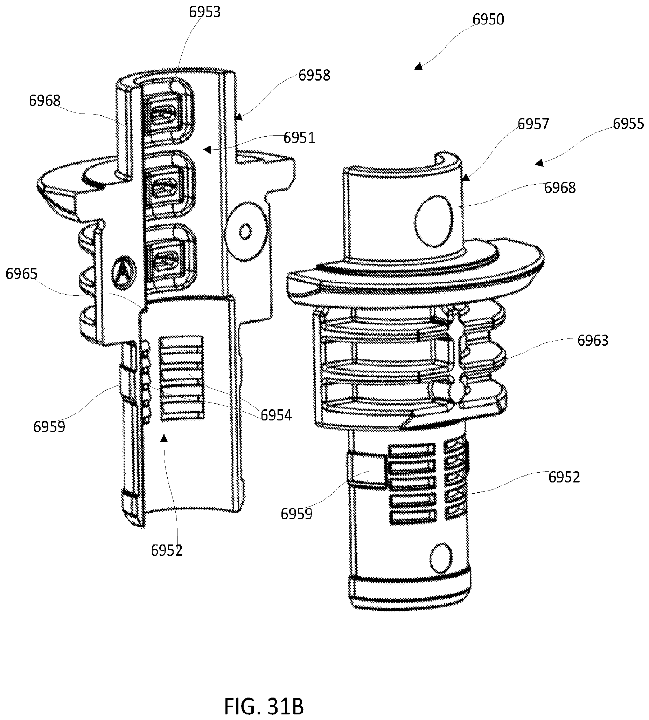

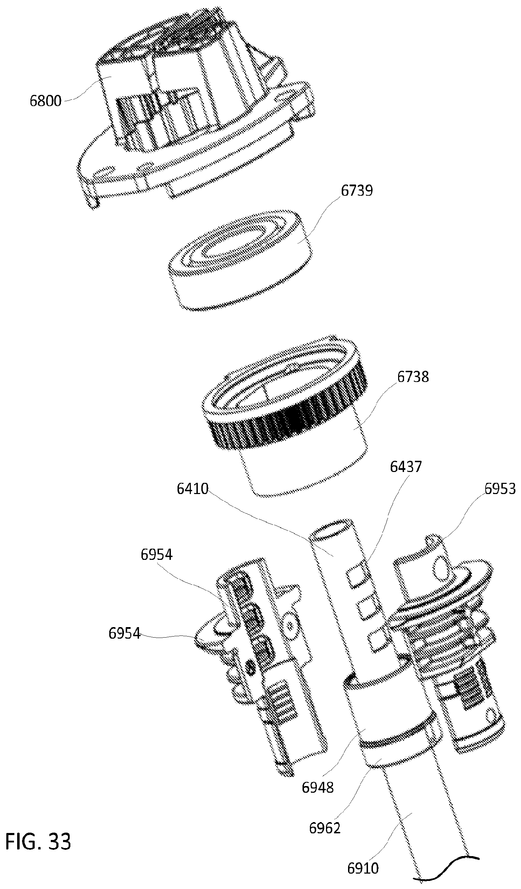

The mechanical structure 6700 includes a flange 6950, and a lock ring 6948. The flange 6950 is coupled to the lower chassis 6762 of the mechanical structure 6700 and a portion of the flange 6950 extends through the opening 6712 of the lower chassis 6762. The flange 6950 includes a proximal end 6955, a distal end 6956, and a centerline CL is defined between the proximal end 6955 and the distal end 6956 (see, e.g., FIGS. 31A and 31B). The flange includes a first portion 6957 and a second portion 6958 (see, e.g., FIGS. 31A, 31B, and 33) that are coupled together along a plane that includes the centerline CL of the flange 6950. The flange 6950 is positioned such that a proximal end portion of the flange 6950 surrounds the shaft 6410 and a distal end portion surrounds the outer cover 6910, as best shown in FIGS. 25, 26A, 27A, and 28. The flange 6950 includes a first coupling portion 6951 and a second coupling portion 6952. The first coupling portion 6951 includes multiple bosses 6953 (see e.g., FIGS. 25, 26A, 28, and 31B) that are received within openings 6437 defined in a wall of the shaft 6410 to couple the flange 6950 to the shaft 6410. When the bosses 6953 are positioned within the openings 6437 of the shaft 6410, movement of the shaft 6410 relative to the flange 6950 in an axial direction is limited.

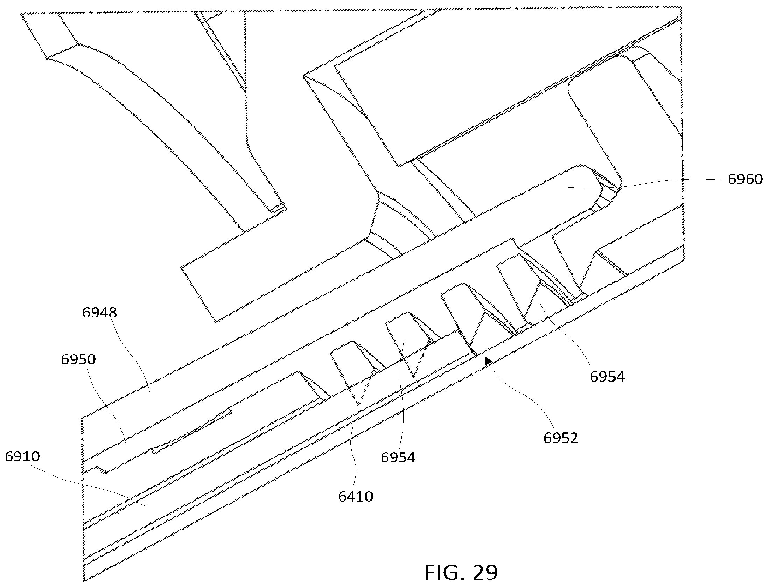

The second coupling portion 6952 couples the flange 6950 to the outer cover 6910 and includes multiple locking teeth 6954 (see FIGS. 29 and 33) that are deformable and engage the proximal end portion 6911 of the outer cover 6910. The lock ring 6948 (see FIGS. 25, 29 and 30) extends at least partially within the opening 6712 of the lower chassis 6762 and is disposed over the distal end portion of the flange 6950 and secured to the flange 6950. More specifically, the lock ring 6948 includes an annular lip portion 6960 that engages and is secured to one or more protrusions 6959 (see, e.g., FIGS. 25, 28, and 30) of the flange 6950. The lock ring 6948 is positioned to urge the locking teeth 6954 to engage the proximal end portion 6911 of the outer cover 6910 to fixedly couple the outer cover 6910 to the shaft 6410. For example, the lock ring 6948 surrounds a portion of the flange 6950, which surrounds the proximal end portion 6911 of the outer cover 6910 such that when the lock ring 6948 is secured to the flange 6950, the lock ring 6948 exerts in inward radial force to be applied to the flange 6950 that causes the locking teeth 6954 of the flange 6950 to deform and press into the material of the outer cover 6910. The lock ring 6948 can optionally include a distal shoulder 6962 (see e.g., FIGS. 9, 24, and 32-34), that has a larger diameter than a diameter of the opening 6712 such that when the lock ring 6948 is positioned around the flange 6950, the larger outer diameter of the distal shoulder 6962 is positioned outside of the lower chassis 6762 and prevents the lock ring 6948 from being installed incorrectly (e.g., being installed backwards) into the opening 6712.

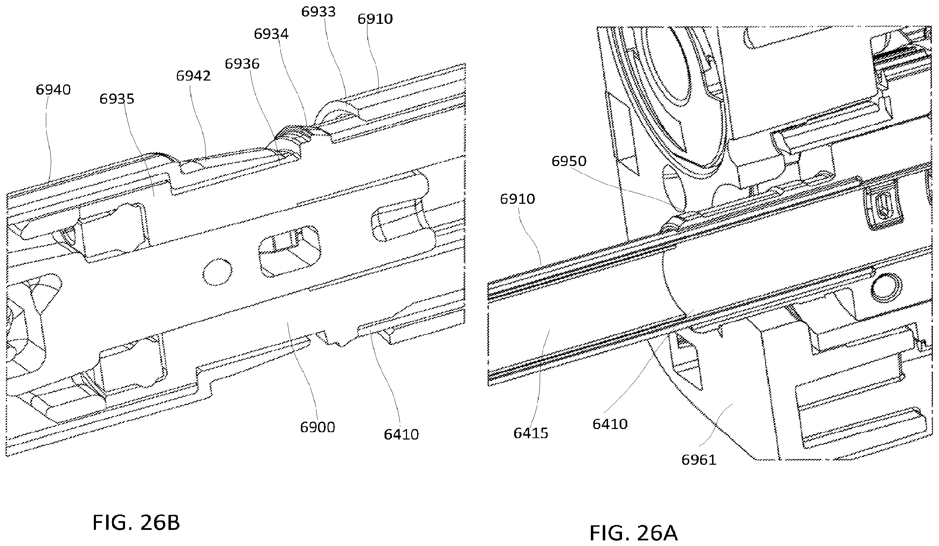

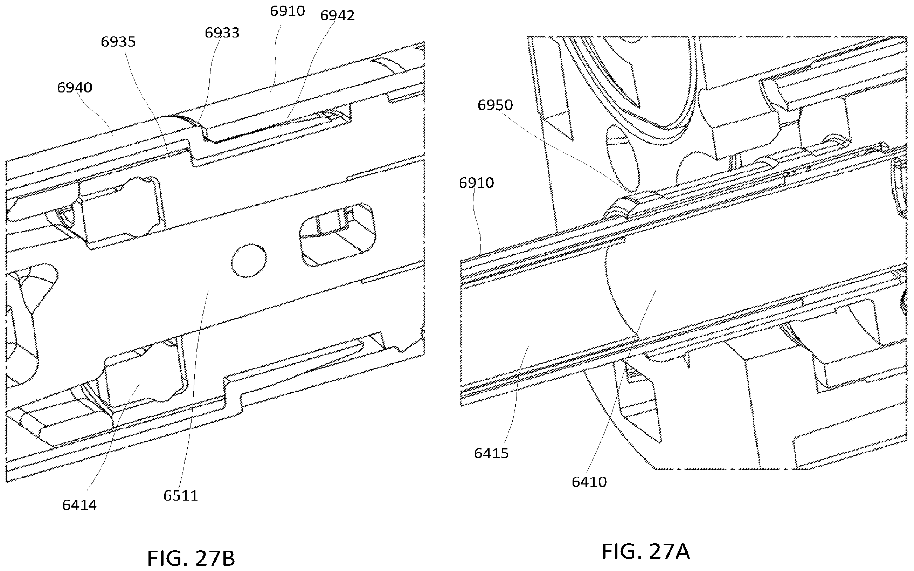

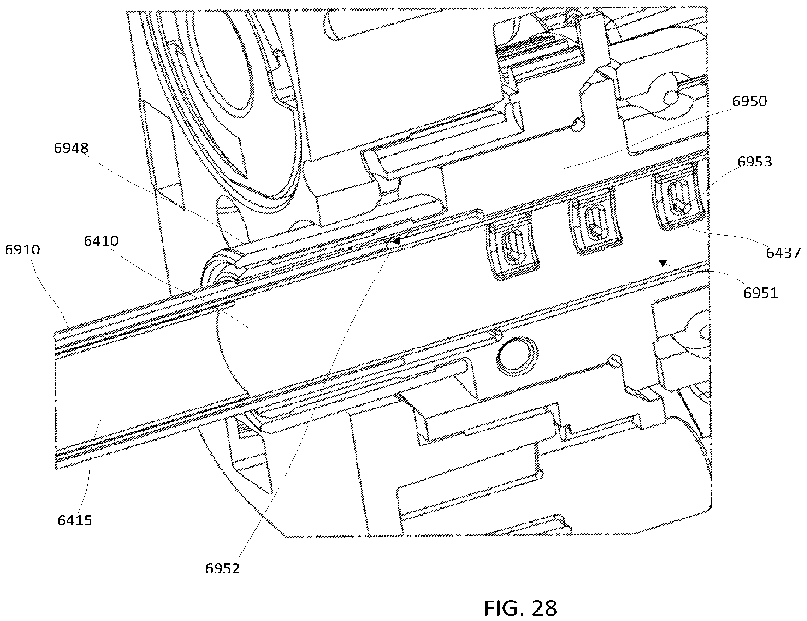

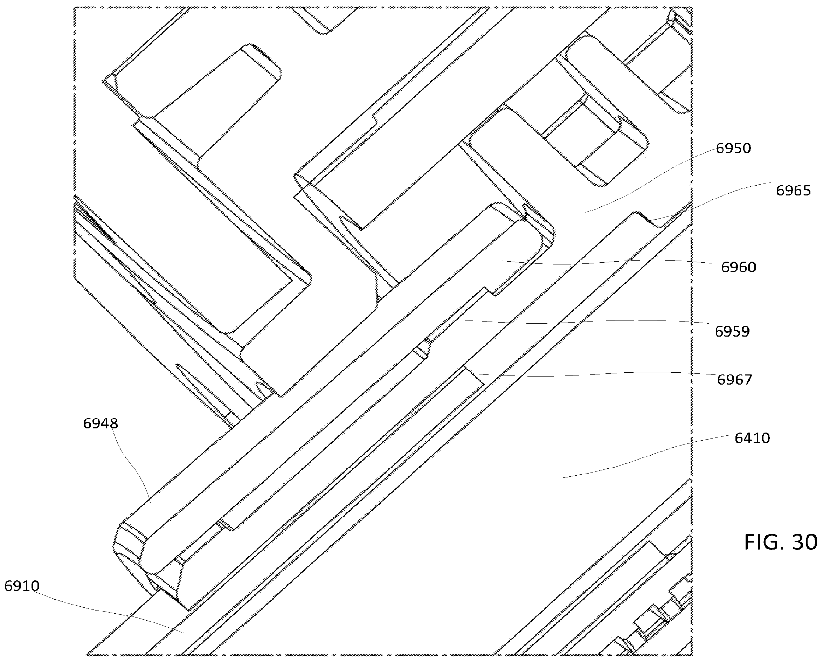

To assemble the medical device 6400 during manufacturing, as described above, the outer cover 6910 can be moved to a retracted position as shown in FIG. 26A. In this position, a proximal end 6914 of the outer cover 6910 engages an inner shoulder 6965 (see, e.g., FIG. 33) of the flange 6950. When the outer cover 6910 is moved to the retracted position, the distal end surface 6933 of the outer cover 6910 is moved to a position proximal of the mounting recess 6936 and first shoulder 6934 of the spacer 6900, as shown in FIG. 26B. With the outer cover 6910 in this position, the coupling protrusion 6942 of the distal tip cover 6940 can be positioned within the mounting recess 6936 of the spacer 6900. After the distal tip cover 6940 is placed in the desired position, the outer cover 6910 can be moved to the advanced position, as shown in FIG. 27A. In the advanced position, a proximal end 6967 of the outer cover 6910 is spaced a distance distally from the inner shoulder 6965 of the flange 6950 and the distal end surface 6933 is engaged with the seal surface 6944 of the distal tip cover 6940 as shown in FIG. 27B. Thus, the mounting protrusion 6942 of the distal tip cover 6940 is trapped between the outer cover 6910 and the spacer 6900 and prevented from being removed. With the outer cover 6910 in the advanced (i.e., distal) position, the lock ring 6948 can be placed through the opening 6712 and the lip portion 6960 of the lock ring 6948 can be secured to the protrusions 6959 of the flange 6950 as described above, and as shown in FIGS. 28 and 30. As described previously when the lock ring 6948 is secured to the flange 6950, the lock ring 6948 urges the locking teeth 6954 of the flange 6900 to press into and engage the inner surface of the outer cover 6910 as shown in FIG. 29. Thus, the lock ring 6948 fixedly couples the outer cover 6910 to the inner shaft 6415 via the flange 6950.

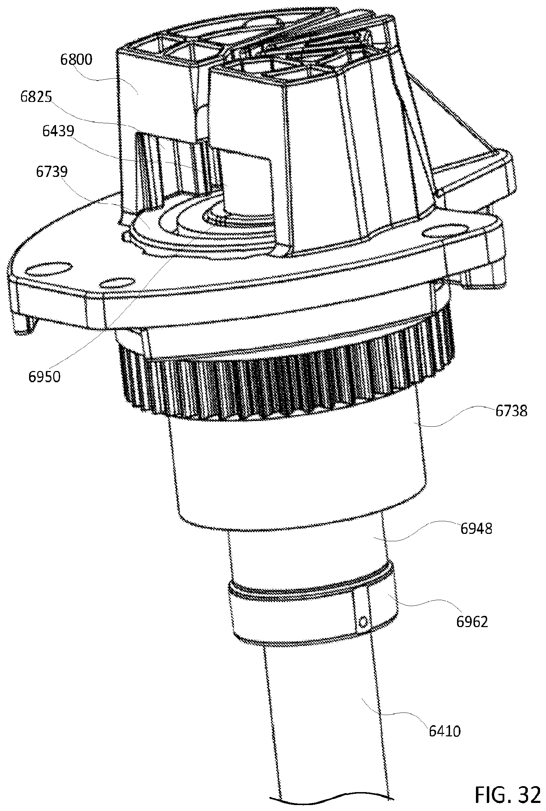

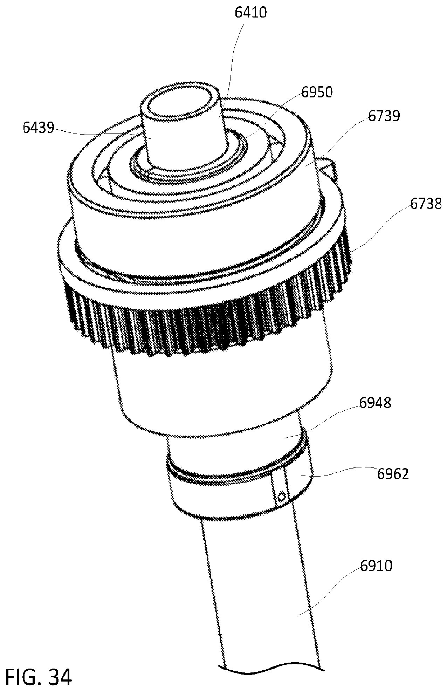

The flange 6950 functions to fixedly couple the outer cover 6910 to the shaft 6410, and to prevent axial movement of the shaft 6410 as described above. The flange 6950 also couples the shaft 6410 to a roll gear 6738 that enables the shaft 6410 to rotate, as shown in FIGS. 32 and 33. More specifically, the flange 6950 includes a roll gear mounting portion 6963 that engages an interior portion of the roll gear 6738 such that the flange 6950 can rotate with the roll gear 6738. With the flange 6950 coupled to the shaft 6410, this also enables the shaft 6410 to rotate with the roll gear 6738 relative to the lower chassis 6762. The flange 6950 also includes a bearing mounting portion 6968 that is coupled within a bearing 6739, and the bearing 6739 is coupled within a cable guide 6800. The bearing mounting portion 6968 is pressed into the bearing 6739 (see e.g., FIG. 34). The cable guide 6800 routes cables from within the shaft 6410 and to the capstans 6710, 6720, 6730, and 6740. The cable guide 6800 can have the same features and function in the same manner as the cable guide described in copending U.S. Provisional Patent Application Serial No. 63/233,904, entitled "Surgical Instrument Cable Control and Routing Structures," the disclosure of which is incorporated herein by reference in its entirety.

As described herein, the medical device 6400 can provide electrical energy to the end effector 6460 to electrically charge the tool members 6462 and 6482 to cut or cauterize tissue. The wrist assembly 6500 may include components formed in part with electrically conductive material to deliver energy to the tool. For example, the first link 6510 and the second link 6610 can be formed with a metallic material. As described above and as shown in FIG. 38, the coupling protrusion of the proximal portion 6511 of the link 6510 includes an interior region 6528 that can receive an electrical connector 6529 configured to receive an electrical wire 6526 to electrically couple the electrical wire 6526 to the proximal link 6511. As described herein, during a procedure using the medical device 6400, it is desirable to prevent or limit fluid contact with the electrically conductive shaft 6410 which acts as an energy shield. The shaft 6410 can become capacitively coupled and charged during the application of energy and to avoid unintended damage to tissue during treatment using the medical device 6400, the distal tip cover 6940, outer cover 6910, spacer 6900, seal 6414 and inner insulative cover 6415 are provided to insulate the shaft 6410 from electrical energy.

The medical device 6400 also includes an energy shield monitoring system (ESM) that can detect if there has been a breach that can cause electrical contact between anatomical tissue and the shaft 6410. For example, the ESM system can detect if there is a breach in the outer cover 6910, inner cover 6415, and/or spacer 6900. If detected, the ESM system can be triggered to turn off the electrical power to the wrist assembly 6500. In some embodiments, the ESM system can be configured the same as or similar to and function the same as or similar to, the systems described in International Application No. PCT/US2018/035436 (Publication No. WO 2018/222899), he disclosure of which is incorporated herein by reference.

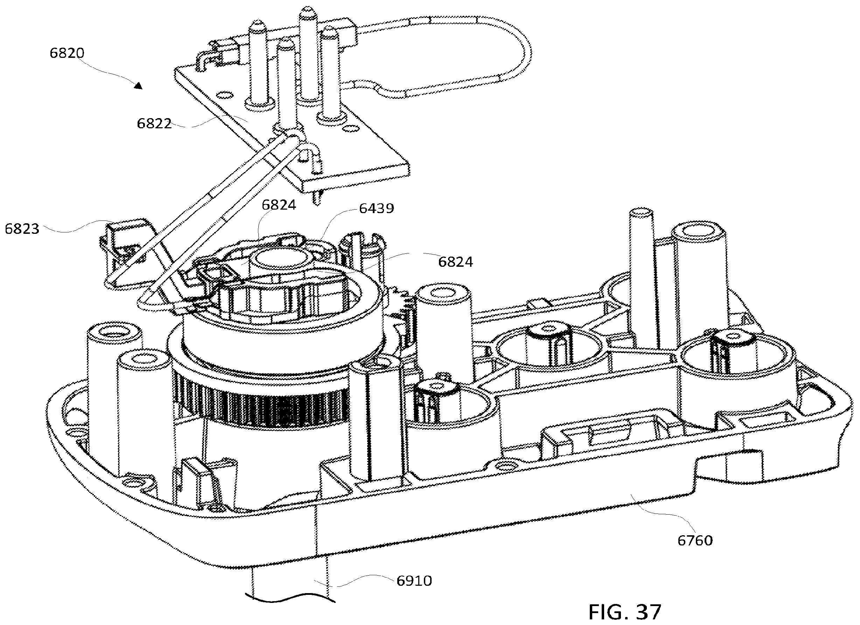

As shown in FIGS. 22 and 35-38, the medical device 6400 includes an electronic circuit 6820 that includes a circuit board 6822, a shaft connector 6823 and shield brushes 6824. The circuit board 6822 can include a processor (not shown) or can be operatively coupled to a processor and can be coupled to the electrical wire 6526 to provide electrical power to the wrist assembly 6500 (see FIG. 38). The shield brushes 6824 are coupled to the circuit board 6822 via electrical wires 6826 and are also coupled to the shaft connector 6823. The shield brushes 6824 are coupled to a proximal end coupling portion 6439 of the shaft 6410 such that the shield brushes 6824 contact the shaft 6410 (see FIG. 37) to electrically couple the shaft 6410 to the circuit board 6822. More specifically, the shield brushes 6824 are coupled to a proximal end of the shaft 6410 that extends proximally of the proximal end of the flange 6950 (see FIGS. 32, 34 and 35). Thus, the shaft 6410 is electrically coupled to the electronic circuit 6820 via the shield brushes 6824.

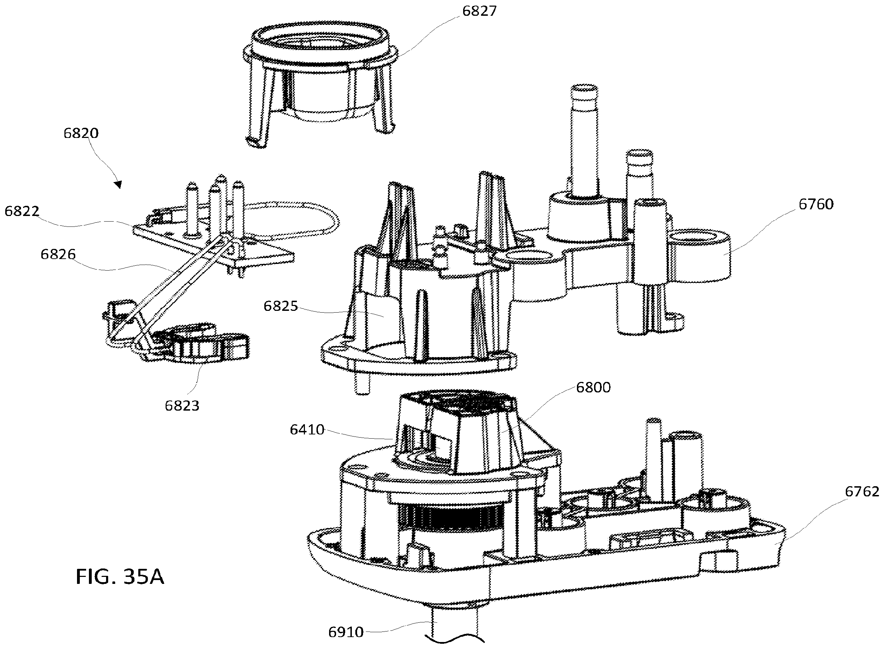

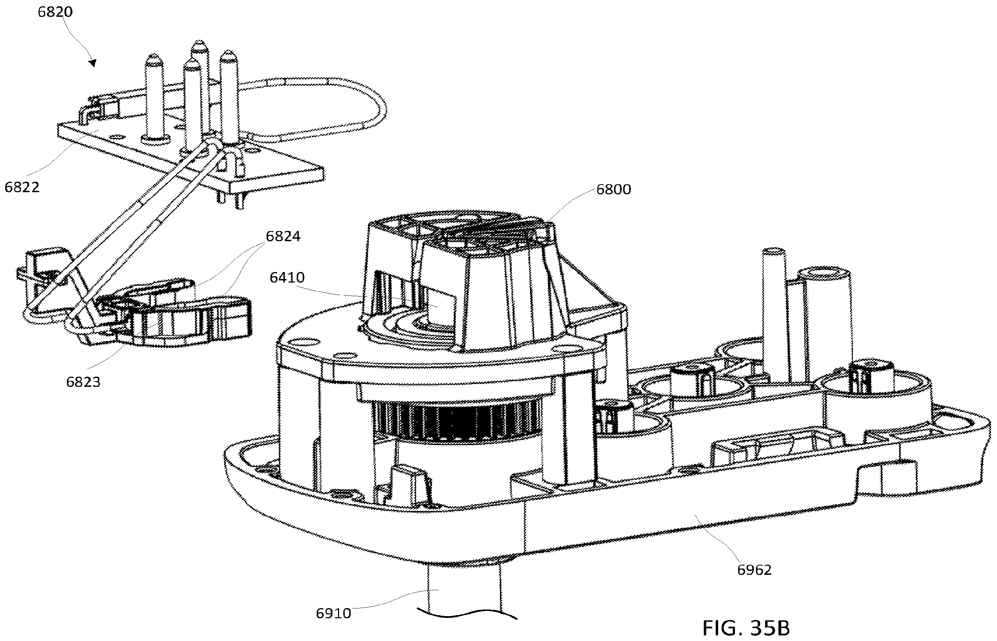

As shown in FIG. 22 and the exploded views of FIGS. 35A and 35B, the upper chassis 6760 is positioned over the cable guide 6800. The cable guide 6800 includes an opening 6825 through which the shaft connector 6823 and shield brushes 6824 can be inserted and coupled to the shaft 6410. The circuit board 6822 is positioned on a top surface of the upper chassis 6760 and is coupled to the shield brushes 6824 via the wires 6826. A housing 6827 is placed over the circuit board 6822 and coupled to the upper chassis 6760.

As described above, the ESM system can detect if there is a breach in the shielding of the shaft 6410, such as a break in the insulative outer cover 6910. More specifically, the processor can receive an input signal associated with a change of impedance of the shaft 6410 via the shield brushes 6824. The change of impedance can indicate, for example, an electrically conductive breach in the outer cover 6910. Based on the input signal. the processor can produce a control signal to turn off the electrical power to the wrist assembly 6500.

As used herein, the term "processor" refers not only to integrated circuits referred to in the art as being included in a computer, but also refers to a controller, a microcontroller, a microcomputer, a programmable logic controller (PLC), an application specific integrated circuit, and other programmable circuits. Additionally, the memory device(s) 1804 may generally comprise memory element(s) including, but not limited to, computer readable medium (e.g., random access memory (RAM)), computer readable nonvolatile medium (e.g., a flash memory), a floppy disk, a compact disc read only memory (CD ROM), a magneto optical disk (MOD), a digital versatile disc (DVD) and/or other suitable memory elements. Such memory device(s) may generally be configured to store suitable computer readable instructions that, when implemented by the processor(s), configure a controller to perform various functions.

While various embodiments have been described above, it should be understood that they have been presented by way of example only, and not limitation. Where methods and/or schematics described above indicate certain events and/or flow patterns occurring in certain order, the ordering of certain events and/or operations may be modified. While the embodiments have been particularly shown and described, it will be understood that various changes in form and details may be made.

For example, any of the instruments described herein (and the components therein) are optionally parts of a surgical assembly that performs minimally invasive surgical procedures, and which can include a manipulator unit, a series of kinematic linkages, a series of cannulas, or the like. Thus, any of the instruments described herein can be used in any suitable surgical system, such as the MIRS system 1000 shown and described above. Moreover, any of the instruments shown and described herein can be used to manipulate target tissue during a surgical procedure. Such target tissue can be cancer cells, tumor cells, lesions, vascular occlusions, thrombosis, calculi, uterine fibroids, bone metastases, adenomyosis, or any other bodily tissue. The presented examples of target tissue are not an exhaustive list. Moreover, a target structure can also include an artificial substance (or non-tissue) within or associated with a body, such as for example, a stent, a portion of an artificial tube, a fastener within the body or the like.

For example, any of the components of a surgical instrument described herein can be constructed from any material, such as medical grade stainless steel, nickel alloys, titanium alloys or the like. Further, any of the links, tool members, tension members, or components described herein can be constructed from multiple pieces that are later joined together. For example, in some embodiments, a link can be constructed by joining together separately constructed components. In other embodiments however, any of the links, tool members, tension members, or components described herein can be monolithically constructed.

Although the instruments are generally shown as having an axis of rotation of the tool members (e.g., axis A1) that is normal to an axis of rotation of the wrist member (e.g., axis A2), in other embodiments any of the instruments described herein can include a tool member axis of rotation that is offset from the axis of rotation of the wrist assembly by any suitable angle.

Although various embodiments have been described as having particular features and/or combinations of components, other embodiments are possible having a combination of any features and/or components from any of embodiments as discussed above. Aspects have been described in the general context of medical devices, and more specifically surgical instruments, but inventive aspects are not necessarily limited to use in medical devices.
The present application also includes the following numbered clauses:
1. A medical device, comprising:
   a shaft, an electrically insulative outer cover, an electrically insulative spacer, and a wrist assembly;
   wherein the shaft comprises an outer surface, an inner surface, and a distal end surface;
   wherein the outer cover is positioned over the outer surface of the shaft and comprises an inner surface and a distal end portion;
   wherein the distal end portion of the outer cover extends beyond the distal end surface of the shaft;
   wherein the spacer comprises a first coupling portion, a second coupling portion, and a circumferential annular protrusion;
   wherein the first coupling portion of the spacer is coupled to the inner surface of the shaft;
   wherein the wrist assembly is coupled to the second coupling portion of the spacer;
   wherein the annular protrusion of the spacer comprises a shoulder and a seal surface;
   wherein the shoulder of the annular protrusion is in contact with the distal end surface of the shaft; and
   wherein the shaft is electrically isolated from electrical energy by the seal surface of the annular protrusion contacting the inner surface of the outer cover.
2. The medical device of clause 1, wherein:
   at least a portion of the shaft between the outer cover and the inner cover is electrically conductive.
3. The medical device of clause 1, wherein:
   the shoulder of the annular protrusion is a first shoulder;
   the spacer comprises a second shoulder and a mounting recess between the annular protrusion and the second shoulder;
   the medical device further comprises a tip cover;
   the tip cover comprises a coupling protrusion and a first seal surface;
   the coupling protrusion is within the mounting recess; and
   fluid is prevented from passing between the tip cover and the spacer by the first seal surface of the tip cover being in contact with the second shoulder of the spacer.
4. The medical device of clause 2, wherein:
   the distal end portion of the outer cover extends over the mounting recess of the spacer to enclose the coupling protrusion of the tip cover;
   the outer cover comprises a distal end surface;
   the tip cover comprises a second seal surface; and
   fluid is prevented from passing between the tip cover and the outer cover by a seal formed between the distal end surface of the outer cover and the second seal surface.
5. The medical device of any of clauses 2-4 wherein:
   the tip cover comprises an inner cover member and an outer cover member;
   the inner cover member is constructed from a first material having a hardness; and
   wherein the outer cover member is constructed from a second material having a hardness; and
   the hardness of the first material is more than the hardness of the second material.
6. The medical device of any of clauses 2-4, wherein:
   the spacer comprises a spacer passageway;
   the second coupling portion of the spacer comprises a plurality of protrusions within the spacer passageway; and
   the wrist assembly is coupled to the spacer by the plurality of protrusions engaging the wrist assembly.
7. The medical device of clause 6, wherein:
   the medical device comprises a cable;
   the spacer comprises a cable opening; and
   the cable is routed from the shaft through the cable opening of the spacer to the wrist assembly.
8. The medical device of clause 6, wherein:
   the wrist assembly comprises a proximal link;
   the proximal link comprises a coupling protrusion; and
   the wrist assembly is coupled to the spacer by the plurality of protrusions of the second coupling portion of the spacer engaging the coupling protrusion.
9. The medical device of clause 6, wherein:
   the medical device comprises an electrically conductive wire;
   the proximal link of the wrist assembly is electrically conductive and comprises a coupling protrusion;
   the coupling protrusion of the proximal link comprises an electrical connector; and
   the electrically conductive wire is received in the electrical connector.
10. The medical device of any of clauses 2-4, wherein:
   the spacer comprises a third coupling portion;
   the medical device further comprises an insulation member adjacent the inner surface of the shaft; and
   the insulation member is coupled to the third coupling portion of the spacer.
11. The medical device of any of clauses 2-4, wherein:
   the spacer comprises an inner surface and a spacer passageway;
   the wrist assembly comprises a coupling protrusion within the spacer passageway;
   the medical device further comprises a cable seal and a cable;
   the cable seal comprises a cable opening and is coupled to the coupling protrusion of the wrist within the spacer passageway to form a seal between the coupling protrusion of the wrist and the inner surface of the spacer; and
   the cable is routed from the shaft, through the cable opening of the cable seal, and to the wrist assembly.
12. The medical device of clause 11, wherein:
   the cable has an untensioned diameter in an untensioned state of the cable; and
   the cable opening has a diameter less than the untensioned diameter of the cable.
13. The medical device of any of clauses 1-4, wherein:
   the medical device further comprises a proximal mechanical structure and an electronic circuit located within the proximal mechanical structure;
   the shaft comprises a proximal end portion coupled to the proximal mechanical structure; and
   the shaft is electrically coupled to the electronic circuit.
14. The medical device of clause 13, wherein:
   the electronic circuit comprises a processor;
   the processor is configured to receive an input signal associated with a change of electrical impedance of the shaft; and
   the processor is configured to produce a control signal on a condition the input signal received by the processor indicates an electrically conductive breach in the outer cover.
15. The medical device of clauses 7, 11 or 12, wherein:
   the cable is a polymeric braided construction.
16. A medical device, comprising:
   a shaft comprising a distal end portion, an outer surface, a first shoulder, a second shoulder, and a mounting recess defined by the outer surface between the first shoulder and the second shoulder;
   an outer cover comprising a distal end surface;
   a wrist assembly coupled to the distal end portion of the shaft; and
   a tip cover comprising an outer surface, a coupling protrusion, a first seal surface, and a second seal surface;
   wherein the outer cover at least partially surrounds the outer surface of the shaft;
   wherein a portion of the tip cover surrounds the wrist assembly; and
   wherein the coupling protrusion of the tip cover is positioned within the mounting recess of the shaft between the outer cover and the shaft such that the first seal surface of the tip cover forms a first seal with the second shoulder of the shaft, and the second seal surface of the tip cover forms a second seal with the distal end surface of the outer cover.
17. The medical device of clause 16, wherein:
   the tip cover comprises an inner cover member and an outer cover member;
   the inner cover member is constructed from a first material having a hardness;
   the outer cover member is constructed from a second material having a hardness; and the hardness of the first material is more than the hardness of the second material.
18. The medical device of any of clauses 16 or 17, wherein:
   the shaft comprises a passageway and a plurality of protrusions within the passageway; and
   the wrist assembly is coupled to the shaft by the plurality of protrusions engaging the wrist assembly.
19. The medical device of clause 18, wherein:
   the medical device comprises a cable;
   the shaft comprises a cable opening; and
   the cable is routed through the passageway of the shaft, through the cable opening of the shaft and to the wrist assembly.
20. The medical device of clause 16, wherein:
   the medical device comprises an electrically conductive wire;
   the wrist assembly comprises an electrically conductive proximal link;
   the proximal link of the wrist assembly comprises a coupling protrusion; and
   the coupling protrusion of the proximal link comprises an electrical connector; and the electrically conductive wire is received in the electrical connector.
21. The medical device of any of clauses 16, 17, 19 or 20, wherein:
   the shaft comprises a coupling portion;
   the medical device further comprises an insulation member adjacent the inner surface of the shaft; and
   the insulation member is coupled to the coupling portion of the shaft.
22. The medical device of any of clauses 16, 17, 19 or 20, wherein:
   the medical device further comprises a proximal mechanical structure and an electronic circuit located within the proximal mechanical structure;
   the shaft comprises a proximal end portion coupled to the proximal mechanical structure; and
   the shaft is electrically coupled to the electronic circuit.
23. The medical device of clause 22, wherein:
   the electronic circuit comprises a processor;
   the processor is configured to receive an input signal associated with a change of electrical impedance of the shaft; and
   the processor is configured to produce a control signal on a condition the input signal received by the processor indicates an electrically conductive breach in the outer cover.
24. The medical device of clause 19, wherein:
   the cable is a polymeric braided construction.
25. A medical device, comprising:
   a shaft comprising a proximal end portion, a distal end portion, an inner surface, and a passageway defined by the inner surface;
   a seal coupled within the passageway of the shaft and comprising a cable seal opening;
   a link coupled to the distal end portion of the shaft;
   a tool member rotatably coupled to the link; and
   a cable comprising a proximal end portion and a distal end portion;
   wherein the proximal end portion of the cable is routed within the passageway of the shaft;
   wherein the distal end portion of the cable is routed through the cable seal opening and is coupled to the tool member;
   wherein movement of the cable causes motion of the tool member;
   wherein the cable seal opening has a diameter;
   wherein the distal end portion of the cable has a first diameter when the cable is in an untensioned state and second diameter when the cable is in a tensioned state;
   wherein the first diameter of the distal end portion of the cable is larger than the diameter of the cable seal opening; and
   wherein the second diameter of the distal end portion of the cable is less than the diameter of the cable seal opening.
26. The medical device of clause 25, wherein:
   the cable is a polymeric braided construction.
27. The medical device of clause 25, wherein:
   at least one of the distal end portion of the cable and a portion of the seal that defines the cable seal opening comprises an oil coating.
28. The medical device of clause 25, wherein:
   at least one of the distal end portion of the cable and a portion of the seal that defines the cable seal opening comprises a hydrophobic material.
29. The medical device of clause 25, wherein:
   the cable seal opening comprises a first taper portion, a second taper portion, and a throat; and
   the throat defines the diameter of the cable seal opening.
30. The medical device of clause 25, wherein:
   a ratio between the first diameter and the second diameter is between 1.2:1 and 1.6:1.
31. The medical device of any of clauses 25-30, wherein:
   the seal defines an outer seal portion that engages and forms a seal with the inner surface of the shaft.
32. The medical device of any of clauses 25-30, wherein:
   the link comprises a coupling protrusion positioned within the passageway of the shaft; and
   the seal includes an inner seal portion that forms a seal within the coupling protrusion of the link.
33. A medical device, comprising:
   a chassis component comprising a distal surface and an opening defined in the distal surface;
   a shaft extending into the opening of the chassis component and coupled to the chassis component;
   an outer cover comprising a proximal end portion;
   a flange positioned at least partially within the chassis component; and
   a lock ring about the flange within the opening of the chassis component;
   wherein the outer cover at least partially surrounds the shaft;
   wherein the proximal end portion of outer cover extends into the opening of the chassis component;
   wherein the flange comprises a shaft coupling portion and a plurality of locking teeth;
   wherein the shaft coupling portion of the flange is coupled to the shaft; and
   wherein the lock ring is positioned to urge the locking teeth to engage the proximal end portion of the outer cover to fixedly couple the outer cover to the shaft.
34. The medical device of clause 33, wherein:
   the flange comprises a proximal end, a distal end, a centerline defined between the proximal end and the distal end, a first portion, and a second portion; and
   the first portion of the flange is coupled to the second portion of the flange along a plane that includes the centerline of the flange.
35. The medical device of claim 33, wherein:
   a portion of the flange that comprises the plurality of locking teeth is deformable.
36. The medical device of clause 33, wherein:
   the shaft comprises a first opening and a second opening;
   the shaft coupling portion of the flange comprises a first boss and a second boss;
   the first boss of the flange is received in the first opening of the shaft; and
   the second boss of the flange is received in the second opening of the shaft.
37. The medical device of clause 33, wherein:
   the medical device further comprises a shaft roll gear;
   the flange comprises a roll gear mounting portion coupled to the roll gear; and
   rotation of the shaft roll gear rotates the shaft and the flange relative to the chassis component.
38. The medical device of clause 37, wherein:
   the medical device further comprises a bearing coupled within the chassis component; and
   the flange comprises a bearing mounting portion coupled to the bearing.
39. The medical device of clause 33, wherein:
   the lock ring comprises a distal portion;
   the distal portion of the lock ring has an outer diameter larger than the opening of the chassis component; and
   the distal portion of the lock ring is outside of the chassis component.

## Claims

1. A medical device, comprising:
a shaft comprising a distal end portion, an outer surface, a first shoulder, a second shoulder, and a mounting recess defined by the outer surface between the first shoulder and the second shoulder;
an outer cover comprising a distal end surface;
a wrist assembly coupled to the distal end portion of the shaft; and
a tip cover comprising an outer surface, a coupling protrusion, a first seal surface, and a second seal surface;
wherein the outer cover at least partially surrounds the outer surface of the shaft;
wherein a portion of the tip cover surrounds the wrist assembly; and
wherein the coupling protrusion of the tip cover is positioned within the mounting recess of the shaft between the outer cover and the shaft such that the first seal surface of the tip cover forms a first seal with the second shoulder of the shaft, and the second seal surface of the tip cover forms a second seal with the distal end surface of the outer cover.

2. The medical device of claim 1, wherein:
the tip cover comprises an inner cover member and an outer cover member;
the inner cover member is constructed from a first material having a hardness;
the outer cover member is constructed from a second material having a hardness; and
the hardness of the first material is more than the hardness of the second material.

3. The medical device of any of claims 1 or 2, wherein:
the shaft comprises a passageway and a plurality of protrusions within the passageway; and
the wrist assembly is coupled to the shaft by the plurality of protrusions engaging the wrist assembly.

4. The medical device of claim 3, wherein:
the medical device comprises a cable;
the shaft comprises a cable opening; and
the cable is routed through the passageway of the shaft, through the cable opening of the shaft and to the wrist assembly.

5. The medical device of claim 1, wherein:
the medical device comprises an electrically conductive wire;
the wrist assembly comprises an electrically conductive proximal link;
the proximal link of the wrist assembly comprises a coupling protrusion; and
the coupling protrusion of the proximal link comprises an electrical connector; and the electrically conductive wire is received in the electrical connector.

6. The medical device of any of claims 1, 2, 4 or 5, wherein:
the shaft comprises a coupling portion and an inner surface;
the medical device further comprises an insulation member adjacent the inner surface of the shaft; and
the insulation member is coupled to the coupling portion of the shaft.

7. The medical device of any of claims 1, 2, 4 or 5, wherein:
the medical device further comprises a proximal mechanical structure and an electronic circuit located within the proximal mechanical structure;
the shaft comprises a proximal end portion coupled to the proximal mechanical structure; and
the shaft is electrically coupled to the electronic circuit.

8. The medical device of claim 7, wherein:
the electronic circuit comprises a processor;
the processor is configured to receive an input signal associated with a change of electrical impedance of the shaft; and
the processor is configured to produce a control signal on a condition the input signal received by the processor indicates an electrically conductive breach in the outer cover.

9. The medical device of claim 4, wherein:
the cable is a polymeric braided construction.
